# EUROPEAN PATENT SPECIFICATION

(11) **EP 3 454 821 B1**
(45) Date of publication and mention of the grant of the patent: **08.11.2023**
(21) Application number: 17724421.7
(22) Date of filing: 12.05.2017
(51) Int. Cl.: A61K 8/02, A61K 8/43, A61K 8/67, A61K 8/86, A61Q 19/00, A61K 9/107, A61K 31/07, A61K 38/28, A61K 47/22

(54) **MICELLES OF D-ALPHA-TOCOPHERYL POLYETHYLENE GLYCOL 1000 SUCCINATE**
MIZELLEN VON D-ALPHA-TOCOPHERYL-POLYETHYLENGLYKOL-1000-SUCCINAT
MICELLES DE SUCCINATE 1000 DE POLYÉTHYLÈNE GLYCOL DE D-ALPHA-TOCOPHÉRYL

(30) Priority: 13.05.2016 GB 201608470
(43) Date of publication of application: 20.03.2019
(60) Divisional application: 23199836.0
(73) Proprietor: Phytoceutical Limited, Midhurst, West Sussex GU29 9DJ (GB)
(72) Inventor: BUCHANAN, Will, Midhurst West Sussex GU29 9DJ (GB); FAIRHURST, David, Aiken SC 29803 (US)
(74) Representative: Barker Brettell LLP
(86) International application number: PCT/GB2017/051336
(87) International publication number: WO 2017/194965

(56) References cited:
- WO-A1-2013/120532
- WO-A1-2013/120532
- CN-A- 103 315 948
- CN-A- 103 315 948
- CN-A- 103 800 913
- US-A1- 2006 073 184
- US-A1- 2006 073 184
- US-A1- 2010 310 642
- US-A1- 2010 310 642
- US-A1- 2013 108 674
- US-A1- 2014 271 593
- MADASWAMY S MUTHU ET AL: "Development of docetaxel-loaded vitamin E TPGS micelles: formulation optimization, effects on brain cancer cells and biodistribution in rats", NANOMEDICINE, vol. 7, no. 3, 1 March 2012 (2012-03-01), pages 353-364, XP055391984, GB ISSN: 1743-5889, DOI: 10.2217/nnm.11.111 cited in the application

## Description

### Field of the invention

The present invention relates to a composition and to a cosmetic formulation comprising the composition. The composition and the cosmetic formulation may find use in the delivery of bio-active therapeutic agents, e.g. vitamins or cosmetic products.

### Background to the invention

One of the major problems faced in micelle production is that micelles which have a high loading capacity are often large and unstable, which places limits on their application particularly for the delivery of cosmetic or therapeutic agents.

Aleksov et al. (2001) (CA 2445478 A1) is a patent specification which discloses Retinol derivatives which potentiate the efficacy of pharmacological activity of a paclitaxel formulation (cytotoxic compound used in cancer treatment), to enable manufacture of a new formulation of paclitaxel with improved solubility, improved storage properties, and increased therapeutic efficacy.

Liu et al. (2009, Chemical Journal of Chinese Universities, 30: 297-301) is a study of decay rates of Retinol and retinyl acetate in various aqueous micellar solutions and at different temperatures. The authors demonstrate that the decay of Retinol and retinyl acetate is much faster in anionic micelles (SDS) than in cationic micelles (CTAB) and neutral micelles (TX-100). The authors also find that the rate constants of the decay of both substances show strong dependence on pH.

Frederick et al. (2014) Liver Disease in Children (4th Edition, Cambridge University Press, UK) provides a description of the intestinal absorption of fat soluble vitamins A, D, E and K. The document discloses that the absorption of fat soluble vitamins is strongly dependent on adequate hepatic secretion of bile acids into the intestinal lumen. Malabsorption of fat-soluble vitamins is common when intraluminal bile acid concentrations are below a critical micelle concentration. The relevant passage describes the use of bile-acid binding agents as a potential therapy for choleostasis and that administration of a liquid containing D-α-Tocopheryl Polyethylene Glycol 1000 Succinate (referred to hereinafter as TPGS or Vitamin E TPGS) and various fat soluble vitamins did not alleviate fat soluble vitamin deficiency in infants.

Omathanu et al. (2012) (US20120219600 A1) is a patent specification which discloses micelle assemblies, compositions having micelle assemblies, and methods for preparing micelle assemblies. The document describes a prolamine (zein) protein conjugated to a polymer, such as a polyethylene glycol (PEG) chain, which conjugates can be used to prepare micelle assemblies and also outlines methods of encapsulating molecules using the conjugates. The resulting micelle assemblies can be used for a variety of applications, such as treating cancer, targeting tumors, reducing the toxicity of a drug *in vivo,* increasing the efficacy of an encapsulated agent *in vivo,* protecting an encapsulated agent against degradation, and enhancing the water solubility of a drug or other agent.

Muthu et al. (2012, Nanomedicine (Lond) 7: 353-364) describes the use of polyethylene glycol (PEG) and vitamin E in the development of docetaxel-loaded D-α-tocopheryl polyethylene glycol 1000 succinate (vitamin E TPGS or TPGS) micelles for the chemotherapy of brain cancer. Vitamin E TPGS combines the advantages of the long half-life of PEG and the high cellular uptake of vitamin E. The TPGS has much lower critical micelle concentration than most phospholipids in micellar formulation, which can be an efficient drug carrier across the blood brain-barrier with high drug encapsulation efficiency, cell uptake, cytotoxicity and desired biodistribution of the formulated drug. TPGS micelles containing docetaxel or coumarin-6 were prepared by the solvent casting method and the direct dissolution method. The document discloses particle sizes of the docetaxel-loaded TPGS micelles between 12 nm and 14 nm and "high", "moderate" and "low" drug-loading levels are claimed.

Mi et al. (2012, Int J Pharm. 438: 98-106) discloses a D-α-tocopheryl polyethylene glycol 1000 succinate (TPGS) prodrug micelle system with cisplatin as a model hydrophilic drug to avoid disadvantages such as low drug loading and low drug encapsulation efficiency due to the drug loss associated with "double emulsion" formulations. The document discloses that TPGS micelles delivered cisplatin with a low critical micelle concentration (CMC) of 5.01 mg/L, a high drug load of 4.95% (w/w) and a pH-responsive drug release kinetics and higher cellular uptake in comparison with the original drug and the TPGS-cisplatin prodrug itself. The TPGS micelle system appears to enhance cisplatin chemotherapy, reducing IC50 values in HepG2 hepatocarcinoma cells, and has the advantage of neuroprotective effects (increased IC50 value for the SH-SY5Y neuroblast-like cells).

Antares Health Products Vitamin E TPGS advertisement (http://www.antareshealthproducts.com/personal care.html) provides a list of the applications of Vitamin E TPGS in personal care and cosmetic products. The document discloses *inter alia* that, in addition to providing a water-soluble form of vitamin E, TPGS serves as an ethanol-free, hypoallergenic, non-irritating emulsifier/excipient for personal care and cosmetic products and that it may be used to solubilize poorly soluble actives.

Papas et al. (2004) (US7790190 B2) is a patent specification which discloses the addition of linoleic acid to the lipid phase of an aqueous emulsion which includes a blend of a therapeutically effective concentration of a lipophile and a concentration of Vitamin E TPGS. The presence of linoleic acid reduces the amount of Vitamin E TPGS that would otherwise be required in the aqueous emulsion by increasing the solubilizing effect of Vitamin E TPGS on the lipophile.

Liang et al. (2013)(CA2537029 C) is a patent specification directed to reverse micellar formulations for the delivery of hydrophobic or lipophilic compounds, particularly therapeutic compounds; comprising: (a) a hydrophilic phase; (b) a hydrophobic continuous phase; and (c) one or more biologically active hydrophobic therapeutic agents.

Huth et al. (2011)(US7923469 B2) is a patent specification which discloses compositions for cleaning and disinfecting contact lenses or for use as eye wash solutions. The compositions described include a liquid aqueous medium and a vitamin derivative component which acts as a surfactant.

Lee et al. (2010) Micellar Nanoparticles Applications for Topical and Passive Transdermal Drug Delivery (http://www.particlesciences.com/docs/Micellar Nanoparticles-Applications for Topical.pdf) is a chapter in the Handbook of Non-Invasive Drug Delivery Systems. It represents a general summary of Micellar Nanoparticle (MNP) technologies and describes the advantages of MNPs, their methods of manufacture and applications in topical and transdermal delivery. MNPs can accommodate both water-soluble and poorly water-soluble active pharmaceutical ingredients (APIs) and present the API in a readily bioavailable form. Combination of MNP technology with transdermal delivery systems can also alleviate hepatic first pass metabolism or degradation in the gastrointestinal tract associated with some drugs. The document reveals that multiphasic nanoemulsions such as MNPs have five basic components: (i) one or more APIs; (ii) solvent; (iii) stabilizer; (iv) oil; and (v) aqueous medium. When these components are mixed together and subjected to a milling process (assisted by high-shear or high-pressure mixing), the API presents in one or more composite fractions (Figure 2.1): solid particulates (micro/nanoparticles), micelle-associated, oil-associated and/or solubilized (in aqueous and/or solvent medium).

WO 2013/120532 A1 discloses an antimicrobial composition containing photochemically stable silver complexes and micelle adducts. In particular, the disclosure relates to a neutral complex of monovalent Silver of formula Ag-L, wherein Ag is an Ag+ ion and L is a ligand of formula 4-mercaptophenylboronic acid, and wherein the silver ion is linked to the mercapto group of the ligand L. Furthermore, the disclosure relates to micelle adducts formed between such complexes and a cationic surfactant.

US 2006/073184 A1 discloses a viscoelastic composition comprising an aqueous solution having a viscoelastic polymer based upon the total volume of the viscoelastic composition. The document also discloses methods of use of the new viscoelastic composition and a packaging device.

Typically, one of the problems faced in micelle production is that micelles which have a high loading capacity are often large, which places limits on their application particularly for the delivery of cosmetic or therapeutic agents. Furthermore, they are often unstable.

It is therefore an object of the invention to generate micelles which are both small and stable but retain a high loading capacity for bio-active agents. The inventors have discovered that using TPGS in the construction of micelles enables the production of micelles which are small and stable but have a high loading capacity for the incorporation of bio-active agents, enabling more efficient bio-active delivery.

### Summary of the invention

The inventors have discovered that using TPGS (both with and without polyhexamethylene biguanide (PHMB) or buffered PHMB) in combination with a bio-active therapeutic agent, in particular an oil-soluble active agent, (for example Retinol) enables the production of micelles that are small and stable but have a high loading capacity for the incorporation of bio-active therapeutic agents, which provides for more efficient bio-active delivery. The addition of PHMB or buffered PHMB enables disinfectant and antiseptic to be added to the micelle delivery as an outer coat which can have benefits in certain applications and additionally act as a preservation mechanism for any formulations comprising said micelles, and additionally allows buffered PHMB or Nanocin, and its known bioactive benefits as a delivery system, to achieve sizes not previously described or known with Nanocin without the invention where Nanocin is in this case an outer coating around the micelle.

Accordingly, in a first aspect the present invention provides a composition comprising micelles of aqueous d-α-tocopheryl polyethylene glycol 1000 succinate (TPGS), polyhexamethylene biguanide (PHMB) or buffered PHMB and a bio-active therapeutic agent; wherein the micelles of the composition do not exceed 31 nm. The composition of the first aspect may be referred to as a micellar composition.

According to a second aspect the present invention provides a cosmetic formulation comprising the composition of the first aspect.

Preferably, the composition of micelles is in the form of a cream, lotion, gel, semi-solid, dispersion, suspension, foam, mousse or spray.

Micelles of the composition may not exceed 30nm, 29nm, 28nm, 27nm, 26nm, 25nm, 24nm, 23nm, 22nm, 21nm, 20nm, 19nm, 18nm, 17nm, 16nm, 15nm, 14nm, 13nm, 12nm, 11 nm, 10nm, 9nm or 8nm. Preferably, micelles of the composition do not exceed 25nm. More preferably, the micelles do not exceed 20nm.

The composition of the claimed invention comprises micelles that include a bio-active therapeutic agent. Micelles comprising TPGS and PHMB may be used to encapsulate a range of bio-active therapeutic agents as cargo. Typically, the composition may comprise more than one bio-active therapeutic agents. Preferably, the bio-active therapeutic agent is an oil-soluble vitamin, which may be provided alone, or in combination with another oil-soluble vitamin.

Preferably, the bio-active therapeutic agent is an oil-soluble active agent. Preferably, the oil-soluble active agent is an oil-soluble vitamin, preferably an oil-soluble aromatic vitamin. For example, the bio-active therapeutic agent may be Retinol, Tocotrienol and/or Tocopherol. The bio-active therapeutic agent may be other oil-soluble active agents, for example, Retinyl Palmitate. Even more preferably, the oil-soluble active agent is Retinol.

In certain embodiments it may be desirable to incorporate more than one bio-active therapeutic agent into the micellar composition. Accordingly, the micellar composition may comprise Retinol and Ascorbyl Palmitate. Where the micellar composition comprises Retinol and Ascorbyl Palmitate, the ratio of Retinol to Ascorbyl Palmitate may be 90:10. Alternatively the ratio of Retinol to Ascorbyl Palmitate may be 99:1, 98:2, 97:3, 96:14, 95:5, 94:6, 93:7, 92:8, 91:9, 89:11, 88:12, 87:13, 86:14, 85:15, 84:16, 83:17, 82:18, 81:19, 80:20, 79:21, 78:22, 77:23, 76:24, 75:25, 74:26, 73:27, 72:28, 71:29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49, 50:50, 49:51, 48:52, 47:53, 46:54, 45:55, 44:56, 43:57, 42:58, 41:59, 40:60, 39:61, 38:62, 37:63, 36:64, 35:65, 34:66, 33:67, 32:68, 31:69, 30:70, 29:71, 28:72, 27:73, 26:74, 25:75, 24:76, 23:77, 22:78, 21:79, 20:80, 19:81, 18:82, 17:83, 16:84, 15:85, 14:86, 13:87, 12:88, 11:89 or 10:90. Preferably, the ratio of Retinol to Ascorbyl Palmitate may be 50:50.

In the micellar composition of the invention the aqueous TPGS may further comprise a water-soluble agent. Preferably, the water-soluble agent is buffered PHMB, for example Nanocin^{™}.

Where the micellar composition comprises Retinol and Ascorbyl Palmitate, the micelles of the composition may not exceed 25nm, 24nm, 23nm, 22nm, 21nm, 20nm, 19nm, 18nm, 17nm, 16nm, 15nm, 14nm, 13nm, 12nm, 11nm, 10nm, 9nm or 8nm. Preferably, micelles of the composition do not exceed 20nm.

Preferably, the aqueous TPGS further comprises a water-soluble agent.

More preferably, the water-soluble agent is PHMB. PHMB may for example, be buffered PHMB (Nanocin^{™}).

Optionally, the water-soluble agent may be, or may further comprise a water-soluble active agent such as insulin, ascorbic acid or L-ascorbic acid.

The present disclosure describes, but does not claim, a method of producing a micellar composition, wherein the micelles do not exceed 100nm, comprising the steps of;
a) dissolving an oil-soluble active agent in an organic solvent to provide a hydrophobic phase;
b) adding the hydrophobic phase into aqueous TPGS; and
c) removing at least a portion of the organic solvent.

Preferably, the hydrophobic phase is added into aqueous TPGS at a concentration in the range 1% to 20%. Alternatively, the hydrophobic phase may be in the range 2% to 19%, 3% to 18%, 4% to 17%, 5% to 16%, 6% to 15%, 7% to 14%, 8% to 13%, 9% to 12%, 2% to 20%, 3% to 20%,4% to 20%,5% to 20%,6% to 20%,7% to 20%,8% to 20%,9% to 20%,10% to 20%,11% to 20%,12% to 20%,13% to 20%,14% to 20%, 15% to 20%,16% to 20%, 17% to 20%, 18% to 20%, 19%, 19.5% or 20%. More preferably, the hydrophobic phase is added into aqueous TPGS at a concentration of 10%.

As noted above, the micelles of the composition of the first aspect include a bio-active therapeutic agent. A range of permissible solvents may be used in conjunction with the methods of the present disclosure to solubilise bio-active agents for encapsulation in micelles depending on the chosen application. Suitable solvents will depend on the bio-active being incorporated into the micellar composition and may be selected accordingly. If it is intended to incorporate only one bio-active in the micellar composition, for example Retinol, then an appropriate solvent will be one in which the chosen bio-active is soluble. Depending on the desired application, a mixture of miscible solvents may be used. Similarly, if it is intended to incorporate more than one bio-active in the micellar composition, for example Retinol and Ascorbyl Palmitate, then an appropriate solvent will be one in which both the chosen bio-actives are soluble. The organic solvent may be a volatile organic solvent and may include, but is not limited to one selected from; GRAS solvents (e.g. Acetic acid, Anisole, Butyl butyrate, 1,3-butylene glycol, Ethanol, Ethyl acetate, Ethyl benzoate, Ethyl butyrate, Ethyl decanoate, Ethyl formate, Ethyl hexanoate, Ethyl lactate, Ethylene dichloride, Glycerin, Glycerol, Glyceryl monooleate, Glyceryl palmitostearate, Isoamyl acetate, Isobutyl acetate, Isopropyl acetate, Isopropyl alcohol, Isopropyl citrate, Lactic acid, Linoleic acid, Methyl acetate, Octanoic Acid, Propionic acid, Propyl acetate, Stearic acid, Water, Ethyl vanillin, Limonene) or ethanol. The organic solvent is preferably an alcohol. More preferably the organic solvent is a C2-C6 carbon chain length alcohol. The organic solvent is preferably ethanol.

Preferably, at least a portion of the volatile organic solvent is removed by subjecting the micellar composition to a vacuum or heating or sonification or a combination thereof.

Preferably, the volatile organic solvent is removed by subjecting the micellar composition to heating and a vacuum. In methods where heating is applied, either alone or in combination with other methods such as application of a vacuum, preferably, the temperature used to remove the volatile organic solvent will be between 50 deg C and 60 deg C.

It is noted that according to this method of micelle formation, if the desired micelle comprises Retinol and Ascorbyl Palmitate at a ratio of 20:80 (Retinol:Ascorbyl Palmitate) and below (i.e. for ratios of 20:80, 19:81, 18:82, 17:83, 16:84, 15:85, 14:86, 13:87, 12:88, 11:89 or 10:90) then step c) of the method; i.e. removal of at least a portion of the organic solvent, may be omitted. At higher ratios, removal of at least a portion of the organic solvent does not prevent the formation of micelles.

The oil-soluble active agent may comprise an oil-soluble vitamin. Preferably, the oil-soluble vitamin is an oil-soluble aromatic vitamin. More preferably, the oil-soluble vitamin is Retinol, Tocotrienol and/or Tocopherol. The oil-soluble active agent may be, for example, Retinyl Palmitate.

Preferably, the micelles do not exceed 30nm, 29nm, 28nm, 27nm, 26nm, 25nm, 24nm, 23nm, 22nm, 21nm, 20nm, 19nm, 18nm, 17nm, 16nm, 15nm, 14nm, 13nm, 12nm, 11nm, 10nm, 9nm or 8nm. Preferably, micelles of the composition do not exceed 25nm. More preferably, the micelles do not exceed 20nm.

In a particularly preferred embodiment, the oil-soluble active agent is Retinol and the micelles do not exceed 20nm.

Preferably, the aqueous TPGS may further comprise a water-soluble agent.

More preferably, the water-soluble agent is PHMB. PHMB may for example, be buffered PHMB (Nanocin^{™}).

The water-soluble agent may be, or further comprises a water-soluble active agent such as insulin, ascorbic acid or L ascorbic acid.

The present disclosure also describes, but does not claim, a method of producing a (reverse) micellar composition, wherein the micelles do not exceed 100nm comprising the steps of;
a) dissolving TPGS into an oil-soluble active agent at a concentration of not more than 20% (w/w) to provide a first solution; and
b) adding an aqueous phase into the first solution at a ratio of not more than 1 part aqueous phase to 4 parts first solution (w/w).

The oil-soluble active agent may be an oil-soluble vitamin. Preferably, the oil-soluble active agent is an oil-soluble aromatic vitamin, for example, including but not limited to, Retinol, Tocopherol or Tocotrienol. The oil-soluble active agent may alternatively comprise an oil-soluble vitamin, for example, Retinyl Palmitate. More preferably, the oil-soluble vitamin is Retinol.

For this method, the amount of TPGS dissolved in the oil-soluble active agent will not exceed 20% (w/w). Preferably, the amount of TPGS dissolved in the oil-soluble active agent will not exceed 10% (w/w). Alternatively, the amount of TPGS dissolved in the oil-soluble active agent may not exceed 19% (w/w), 18% (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1 % (w/w), 0.5% (w/w). More preferably, the amount of TPGS that may be added into the oil-soluble active agent will not exceed 5% (w/w).

The amount of oil soluble active agent that TPGS may be dissolved in may not exceed 19% (w/w), 18% (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1% (w/w) or 0.5% (w/w).

Optionally, the aqueous phase comprises a water-soluble active agent. The water-soluble active agent may be for example PHMB, buffered PHMB (Nanocin^{™}), insulin, ascorbic acid or L-ascorbic acid. Preferably, the water-soluble active agent is PHMB or buffered PHMB (Nanocin^{™}).

For this method, the amount of aqueous phase that is added into the first solution will not exceed 20% (w/w). The aqueous phase is added into the first solution at a ratio of not more than 1 part aqueous phase to 4 parts first solution (w/w). More preferably, amount of aqueous phase that may be added into the first solution is 10% (w/w). Alternatively, amount of aqueous phase that may be added into the first solution may not exceed 19% (w/w), 18 % (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1% (w/w), 0.5% (w/w).

Optionally, the oil-soluble vitamin may be Retinol. Where the oil-soluble vitamin is Retinol, the micelles may not exceed 25nm. Alternatively, micelles of the composition where the oil-soluble vitamin is Retinol may not exceed 24nm, 23nm, 22nm, 21nm, 20nm, 19nm, 18nm, 17nm, 16nm, 15nm, 14nm, 13nm, 12nm, 11nm, 10nm, 9nm, 8nm, 7nm, 6nm or 5nm. Preferably, where the oil-soluble vitamin is Retinol, micelles of the composition do not exceed 25nm. More preferably, where the oil-soluble vitamin is Retinol, the micelles do not exceed 20nm. More preferably, where Retinol is provided in the outer phase and buffered PHMB (Nanocin^{™}) in the inner, water phase the micelles do not exceed 25nm.

Furthermore, the oil-soluble active agent may be Retinol and may itself be provided as a (reverse) micelle, for example water or a water-soluble active agent encapsulated by Retinol and/or TPGS.

Optionally, the aqueous phase comprises a water-soluble active agent. The water-soluble active agent may be for example PHMB, buffered PHMB (Nanocin^{™}), insulin or ascorbic acid. Preferably, the water-soluble active agent is PHMB or buffered PHMB (Nanocin^{™}). Preferably, the micelles do not exceed 25nm. Alternatively, micelles of the composition may not exceed 24nm, 23nm, 22nm, 21nm, 20nm, 19nm, 18nm, 17nm, 16nm, 15nm, 14nm, 13nm, 12nm, 11nm, 10nm, 9nm, 8nm, 7nm, 6nm or 5nm. Preferably, where the oil-soluble vitamin is Retinol, micelles of the composition do not exceed 25nm. More preferably, where the oil-soluble vitamin is Retinol, the micelles do not exceed 20nm.

The present disclosure also describes, but does not claim, a method of making a micellar composition comprising the steps of;
a) providing a micellar composition with an outer hydrophilic phase; and
b) adding the micellar composition with an outer hydrophilic phase into a solution comprising TPGS solubilised in an oil soluble vitamin.

Advantageously, the composition is a double micellar composition that is provided by providing a micellar composition with an outer hydrophilic phase; and adding the micellar composition with an outer hydrophilic phase into a solution comprising TPGS solubilised in an oil soluble vitamin, for example Retinol, Tocotrienol or Tocopherol.

The micellar composition with an outer hydrophilic phase may optionally be obtained by;
a) dissolving an oil-soluble active agent in an organic solvent to provide a hydrophobic phase;
b) adding the hydrophobic phase into aqueous TPGS; and
c) removing at least a portion of the organic solvent.

Optionally, the oil-soluble vitamin may be Retinol and may itself be provided as a micelle comprising Retinol dissolved in TPGS.

Furthermore, the oil-soluble active agent may be Retinol and may itself be provided as a (reverse) micelle, for example water or a water-soluble active agent encapsulated by Retinol and/or TPGS. For instance, where TPGS may be dissolved in Retinol, followed by the addition of water where TPGS is 0-20% (w/w) and water is 0-20% (w/w). The aqueous phase may comprise Nanocin^{™} or buffered PHMB. TPGS is preferably 10% (w/w). Water is preferably 10% (w/w) or 5% (w/w).

The present disclosure also describes, but does not claim, a method of making a micellar composition comprising the steps of;
a) providing a micellar composition with an outer oil phase; and
b) adding the micellar composition with an outer oil phase into aqueous TPGS, at a ratio of not more than 1 part micellar composition with outer oil phase to 4 parts aqueous TPGS (w/w).

Preferably, the micellar composition with an outer oil phase will comprise TPGS, PHMB and Retinol. Alternatively, the micellar composition with outer oil phase will comprise TPGS, Retinol and water.

Preferably, the amount of micellar composition with outer oil phase that may be added into aqueous TPGS will not exceed 20% (w/w). More preferably, amount of micellar composition with outer oil phase that may be added into the aqueous TPGS is 10% (w/w). Alternatively, amount of micellar composition with outer oil phase that may be added into the aqueous TPGS may not exceed 19% (w/w), 18% (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1% (w/w), 0.5% (w/w).

The present disclosure also describes, but does not claim, a method of producing a micellar composition, wherein the micelles do not exceed 100nm, and preferably not exceeding 25nm, comprising the steps of;
a. dissolving an oil-soluble active agent into aqueous TPGS at a concentration of not more than 20% (w/w) to provide a first solution;
b. dissolving TPGS into an oil soluble vitamin to provide a second solution; and
c. adding the first solution into the second solution at a ratio of not more than 1 part first solution aqueous phase to 4 parts second solution (w/w).

Optionally, the oil-soluble active agent may be an oil-soluble vitamin, preferably an oil-soluble aromatic vitamin. The oil-soluble active agent may be Retinol, Tocotrienol and/or Tocopherol. Alternatively, the oil-soluble active agent may be, for example, Retinyl Palmitate. Even more preferably, the oil-soluble active agent is Retinol. For this method, in providing the first solution, the amount of oil-soluble active agent that may be dissolved into aqueous TPGS will not exceed 20% (w/w). Preferably, the amount of oil-soluble active agent that may be dissolved into aqueous TPGS will not exceed 10% (w/w). Alternatively, the amount of oil-soluble active agent that may be dissolved into aqueous TPGS may not exceed 19% (w/w), 18% (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1% (w/w), 0.5% (w/w). More preferably, the amount of oil-soluble active agent that may be dissolved into aqueous TPGS will not exceed 5% (w/w).

For this method, in providing the second solution, the amount of TPGS that may be dissolved into an oil-soluble vitamin will not exceed 20% (w/w). Preferably, the amount of TPGS dissolved in the oil-soluble active vitamin will not exceed 10% (w/w). Alternatively, the amount of TPGS dissolved in the oil-soluble vitamin may not exceed 19% (w/w), 18% (w/w), 17% (w/w), 16% (w/w), 15% (w/w), 14% (w/w), 13% (w/w), 12% (w/w), 11% (w/w), 10% (w/w), 9% (w/w), 8% (w/w), 7% (w/w), 6% (w/w), 5% (w/w), 4% (w/w), 3% (w/w), 2% (w/w), 1% (w/w), 0.5% (w/w). More preferably, the amount of TPGS that may be added into the oil-soluble vitamin will not exceed 5% (w/w).

For this method, the first solution (comprising oil-soluble active agent dissolved into aqueous TPGS) is added into the second solution (comprising TPGS dissolved into an oil-soluble vitamin) at a ratio of not more than 1 part first solution aqueous phase to 4 parts second solution. Preferably the amount of first solution that may be added into the second solution may not exceed 20% (w/w), 19% (w/w), 18% (w/w), 17% (w/w), 16% (w/w), 15% (w/w), 14% (w/w), 13% (w/w), 12% (w/w), 11% (w/w) or 10% (w/w). Preferably, the amount of first solution that may be added into the second solution may not exceed 10% (w/w), 9% (w/w), 8% (w/w), 7% (w/w), 6% (w/w), 5% (w/w), 4% (w/w), 3% (w/w), 2% (w/w), 1% (w/w), 0.5% (w/w).

The present disclosure enables small, stable micelles with high loading capacity to be produced by the use of a bio-active therapeutic agent, in particular an oil-soluble active agent (for example Retinol) in conjunction with TPGS (with and without PHMB or buffered PHMB or Nanocin).

The loading capacity of micelles is measured as w/w of TPGS and the bio-active therapeutic agent. In the case of micelles "high loading capacity" is in the range 0.1 - 20% inner oil phase. Accordingly, loading capacity may be in the range 0.1 - 20% (w/w), 1 - 20% (w/w), 2 - 20% (w/w), 3 - 20% (w/w), 4 - 20% (w/w), 5 - 20% (w/w), 6 - 20% (w/w), 7 - 20% (w/w), 8 - 20% (w/w), 9 - 20% (w/w), 10 - 20% (w/w), 11 - 20% (w/w), 12 - 20% (w/w), 13 - 20% (w/w), 14 - 20% (w/w), 15 - 20% (w/w), 16 - 20% (w/w), 17 - 20% (w/w), 18 - 20% (w/w) or 19 - 20% (w/w). Alternatively, loading capacity of the micelles may be in the range 0.1 - 20% (w/w), 1 - 19% (w/w), 1 - 18% (w/w), 1 - 17% (w/w), 1 - 16% (w/w), 1 - 15% (w/w), 1 - 14% (w/w), 1 - 13% (w/w), 1 - 12% (w/w), 1 - 11% (w/w), 1 - 12% (w/w), 1 - 11% (w/w), 1 - 10% (w/w), 1 - 9% (w/w), 1 - 8% (w/w), 1 - 7% (w/w), 1 - 6% (w/w), 1 - 5% (w/w), 1 - 4% (w/w), 1 - 3% (w/w) or 1 - 2% (w/w). Preferably, loading capacity of the micelles may be in the range 1 - 20% (w/w), 2 - 19% (w/w), 3 - 18% (w/w), 4 - 17% (w/w), 5 - 16% (w/w), 6 - 15% (w/w), 7 - 14% (w/w), 8 - 13% (w/w), 9 - 12% (w/w), 10 - 11% (w/w).

This is similar in the case of reverse micelles generated in accordance with the disclosure, where the outer oil phase is a bio-active therapeutic agent, in particular an oil-soluble active agent (for example Retinol), and the inner phase is an aqueous phase (for example Nanocin or water-soluble bio-active), the loading capacity of the micelles (inner aqueous phase). The loading capacity of reverse micelles is measured as w/w of TPGS and the bio-active therapeutic agent. In the case of reverse micelles "high loading capacity" is in the range 0.1 - 20% inner water phase. Accordingly, loading capacity may be in the range 0.1 - 20% (w/w), 1 - 20% (w/w), 2 - 20% (w/w), 3 - 20% (w/w), 4 - 20% (w/w), 5 - 20% (w/w), 6 - 20% (w/w), 7 - 20% (w/w), 8 - 20% (w/w), 9 - 20% (w/w), 10 - 20% (w/w), 11 - 20% (w/w), 12 - 20% (w/w), 13 - 20% (w/w), 14 - 20% (w/w), 15 - 20% (w/w), 16 - 20% (w/w), 17 - 20% (w/w), 18 - 20% (w/w) or 19 - 20% (w/w). Alternatively, loading capacity of the reverse micelles may be in the range 0.1 - 20% (w/w), 1 - 19% (w/w), 1 - 18% (w/w), 1 - 17% (w/w), 1 - 16% (w/w), 1 - 15% (w/w), 1 - 14% (w/w), 1 - 13% (w/w), 1 - 12% (w/w), 1 - 11% (w/w), 1 - 12% (w/w), 1 - 11% (w/w), 1 - 10% (w/w), 1 - 9% (w/w), 1 - 8% (w/w), 1 - 7% (w/w), 1 - 6% (w/w), 1 - 5% (w/w), 1 - 4% (w/w), 1 - 3% (w/w) or 1 - 2% (w/w). Preferably, loading capacity of the reverse micelles may be in the range 1 - 20% (w/w), 2 - 19% (w/w), 3 - 18% (w/w), 4 - 17% (w/w), 5 - 16% (w/w), 6 - 15% (w/w), 7 - 14% (w/w), 8 - 13% (w/w), 9 - 12% (w/w), 10 - 11% (w/w).

### Brief description of the figures

The present disclosure will now be described in detail with reference to examples and with reference to the accompanying drawings, in which:
**Figure 1** shows the size of nanoparticles of Retinol dissolved in absolute ethanol with different concentrations of Nanocin^{™}.
**Figure 2** shows micelles with TPGS, Retinol and Nanocin^{™} showing nano-size. Panel A shows the size of nanoparticles of 5% micellar solution of Retinol with different concentrations of Nanocin^{™}.Panel B shows results for 5% micellar Retinol without Nanocin^{™} (both with TPGS).
**Figure 3** shows a comparison of particle concentration on the incubation of Retinol and Nanocin^{™} (in ethanol) after 2 days at 4°C with, or without warming to 37°C before reading.
**Figure 4** shows the particle size distribution for 1:3 Retinol: Nanocin^{™} in ethanol.
**Figure 5** shows the kinetics absorbance of nanoparticles of Retinol dissolved in absolute ethanol with different concentrations of Nanocin^{™} during 24 h storage (red line - 10 mg/ml Retinol alone, blue line - 10 mg/ml Retinol with 10 mg/ml Nanocin^{™}, green line - 10 mg/ml Retinol with 50 mg/ml Nanocin^{™} and brown line - 10 mg/ml Retinol with 100 mg/ml Nanocin^{™}). X axis - time (S); Y axis - kinetics absorbance (405nm).
**Figure 6** shows particle concentration (e8) with Retinol (RA) and Nanocin^{™} in Propylene Glycol (PEG in tables or PG).
**Figure 7** shows modal particle sizes of Retinol and Nanocin^{™} (in PEG in tables or PG) after 2 days at 4°C with and without warming to 37°C.
**Figure 8** shows the particle size distribution for 1:5 Retinol: Nanocin^{™} in Propylene Glycol (PEG in tables or PG).
**Figure 9** shows the kinetics absorbance of nanoparticles of Retinol dissolved in propylene glycol with different concentrations of Nanocin^{™} during 24 h storage (red line - 10 mg/ml Retinol alone, blue line - 10 mg/ml Retinol with 10 mg/ml Nanocin^{™}, green line - 10 mg/ml Retinol with 50 mg/ml Nanocin^{™} and brown line - 10 mg/ml Retinol with 100 mg/ml Nanocin^{™}). X axis - time (S); Y axis - kinetics absorbance (405nm).
**Figure 10** shows a comparison of particle concentration after incubation of aqueous Nanocin^{™} with Vitamin C (in water) (with no oil or TPGS) after 3 hours or 4 days at 4°C.
**Figure 11** shows particle concentrations (minus their control values) produced after 4 days at 4°C with Vitamin C and Nanocin^{™} at different ratios.
**Figure 12** shows the modal particle size of nanoparticles after incubation of Vitamin C and Nanocin^{™} (in water) over 4 days at 4°C.
**Figure 13** shows the particle size distribution for 1:5 Vitamin C:Nanocin^{™} mixture.
**Figure 14** shows the absorbance spectra of Vitamin C (1%) dissolved in water without Nanocin^{™} (red line) and with 1% Nanocin^{™} (blue line) and 7% Nanocin^{™} (green line) after 2 days storage.
**Figure 15** shows the absorbance spectra of Vitamin C (1%) dissolved in water without Nanocin^{™} (red line) and with 1% Nanocin^{™} (blue line) and 7% Nanocin^{™} (green line) after 6 days storage.
**Figure 16** shows the kinetics absorbance of Vitamin C (1%) dissolved in water without Nanocin^{™} (red line) and with 1% Nanocin^{™} (blue line) and 7% Nanocin^{™} (green line) concentrations during 6 days storage.
**Figure 17** shows DLS measurements for micelles of Tocopherol with TPGS using method of Example 7.
**Figure 18** shows micelles of Tocotrienol according to Example 7.
**Figure 19** shows non-formation of micelles comprising soy oil.
**Figure 20** shows 5% TPGS in Retinol (lipophilic) with Nanocin^{™} added in to form reverse micelle.
**Figure 21** shows TPGS in Retinol only reverse micelle.
**Figure 22** shows Micellar Retinol (TPGS) no Nanocin^{™} with 5% Ascorbic Acid in outer aqueous phase, low pH around 3.
**Figure 23** shows Micellar Retinol with TPGS and insulin in outer aqueous phase.
**Figure 24** shows Micellar Retinol with TPGS and Nanocin^{™} and 200 UL Ascorbic Acid in outer aqueous solution.
**Figure 25** shows Micellar Retinol with TPGS and Nanocin^{™} with 200µL insulin in outer aqueous phase.
**Figure 26** shows measurements for Ascorbyl Palmitate micelles with TPGS without Nanocin. For 100% Ascorbyl Palmitate as active no micelles seen just precipitate. For 80% Ascorbyl Palmitate and 20% Retinol together in ethanol showed the following results, larger particles and some cloudiness, ethanol being driven off produced precipitate.
**Figure 27** shows Micellar TPGS Ascorbyl Palmitate with 50:50% Retinol.
**Figure 28** shows Micellar TPGS with 10% Ascorbyl Palmitate and 90% Retinol in inner oil phase.
**Figure 29** **shows** TPGS micelles with retinol at 0 days storage.
**Figure 30** **shows** TPGS micelles with retinol after 1 year.
**Figure 31** **shows** TPGS micelles with retinol and Nanocin^{™} at 0 days storage.
**Figure 32** **shows** TPGS micelles with retinol and 2% Nanocin^{™} and after 1 year.
**Figure 33** **shows** TPGS micelles with retinol and 5% Nanocin^{™} and after 1 year.
**Figure 34** **shows** TPGS micelles with retinol and 10% Nanocin^{™} after 1 year.

### Detailed description of the invention

The scope of the claimed invention is defined by the claims. The following nonlimiting examples are provided to illustrate the invention:

### Example 1. Micellar suspension of Retinol (MicRet)

A micellar suspension of Retinol was obtained by the following method:
1. Prepare a 5% stock solution of Retinol in ethanol (EtOH) by adding 1g of Retinol into 19g EtOH using a magnetic stirrer. If 5% exceeds the solubility limit the adjust accordingly. Ideally, the maximum concentration of Retinol in EtOH should be obtained.
2. Prepare a 10% stock solution of Vitamin E-TPGS in water. If the TPGS is supplied as a 20% aqueous solution, then simply dilute. If supplied as a solid material then add 10g of the TPGS to 90g H₂O. Follow the instructions on the data sheet for preparation of a solution.
3. Measure the particle size/size distribution.
4. Set up a magnetic stirrer (or paddle stirrer) and hotplate together with a small sonic probe/homogenizer. Place 20g of the TPGS stock solution in a beaker. Commence fairly vigorous stirring. Raise the temperature to approximately 50 deg C, but less than 60 deg C. Check that the TPGS does not appear to "cloud out". It should not, but if so, reduce the temperature until solution is clear. Quickly add in the 20g of the stock Retinol solution with homogenization and constant stirring and constant temperature to "flash off" (evaporate) all the EtOH. This step is aided by vacuum if available. Continue until no evidence of EtOH. Cool quickly to 40 deg C with constant stirring. Bottle. If possible, store under N₂. If not, fill container as much as possible. Upon removal of the EtOH, Retinol will begin to precipitate out and so must immediately be taken up into the TPGS micellar suspension.
5. Measure the particle size/distribution.

To make micellar Retinol with Nanocin^{™} the following additional method was used:

### MicRet:Nanocin^{™} Combination preparation.

General steps to prepare MicRet:Nanocin^{™} Combination:
- **Pipette appropriate MicRet¹ to tube**
- **Add appropriate amount of Nanocin² (20% solution) and mix well by pipetting**
- **Add water³ to compensate volume and mix well by pipetting**

1 - keep the same amount of MicRet in different MicRet:Nanocin^{™} Combination
2 - appropriate amount of Nanocin^{™} (20%) to obtain required concentration of Nanocin
3 - compensate volume of preparing MicRet-Nanocin^{™} Combination with water

**Table 1. Example for preparing 10 ml of: MicRet:10%Nanocin, MicRet:5%Nanocin^{™} and MicRet:2%Nanocin^{™}.**

| MicRet | 20% Nanocin | Water (to compensate volume up to 10 ml) | Obtained MicRet:Nanocin^{™} Combination |
|---|---|---|---|
| 5 ml | 5 ml | 0 ml | **MicRet:10% Nanocin** |
| 5 ml | 2.5 ml | 2.5 ml | **MicRet:5% Nanocin** |
| 5 ml | 1 ml | 4 ml | **MicRet:2% Nanocin** |

The above method keeps the same level of MicRet in all combinations.

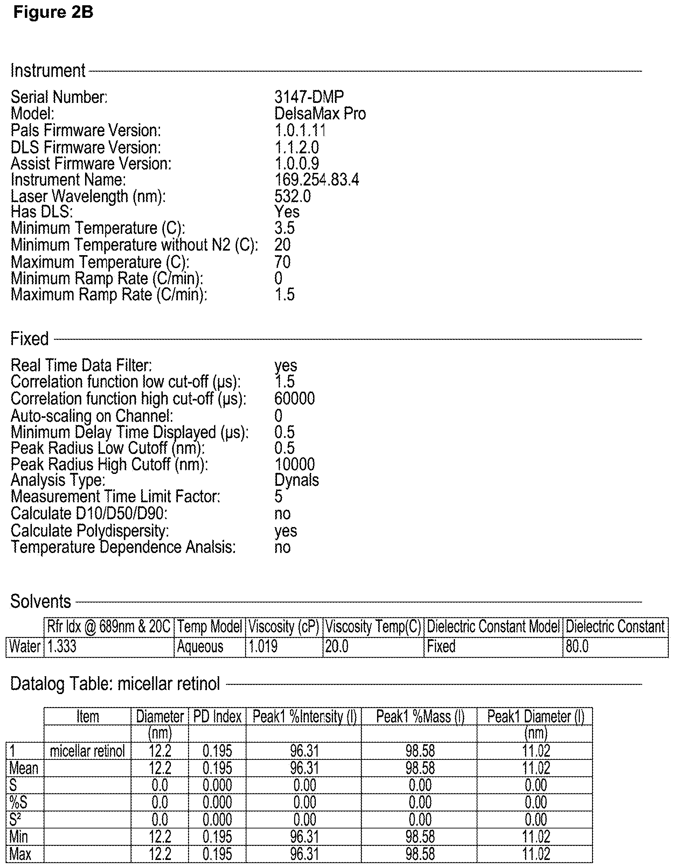

The micellar composition without Nanocin^{™} thus obtained was analysed by DLS (see **Figure 2B**) and found to be stable using freeze thaw stability (see **Table 2**) over a period of 3 days

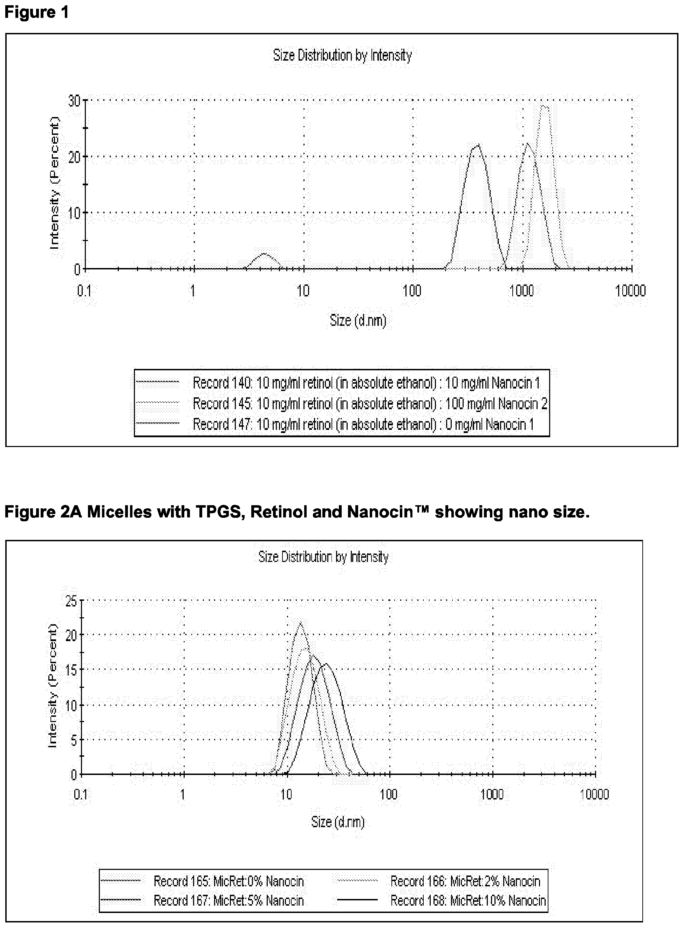

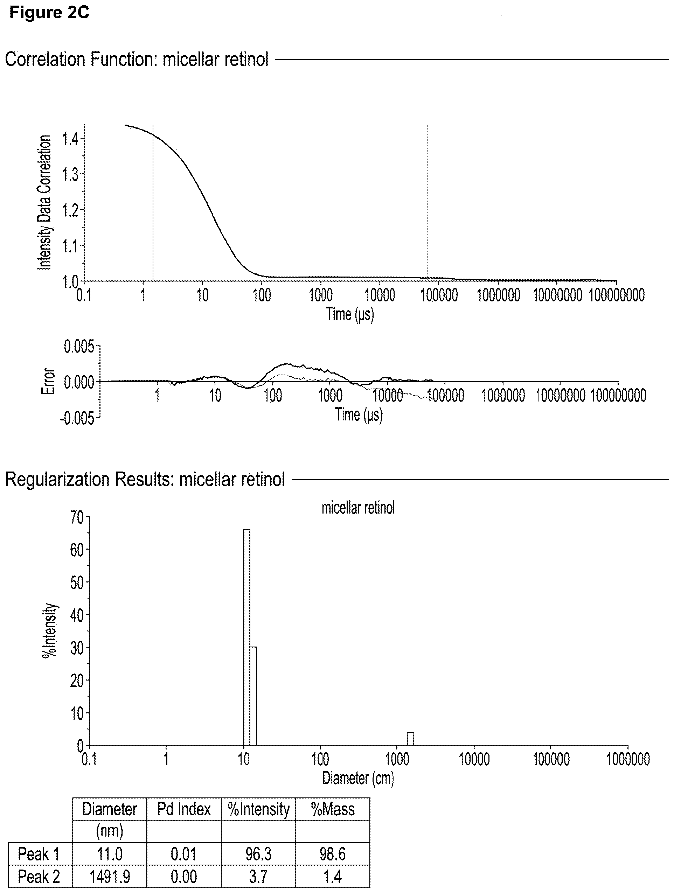

Furthermore, micelles comprising buffered PHMB or Nanocin^{™} and TPGS, and Retinol were found to be equally stable and slightly larger size around 30nm in this case (see **Figure 2**).

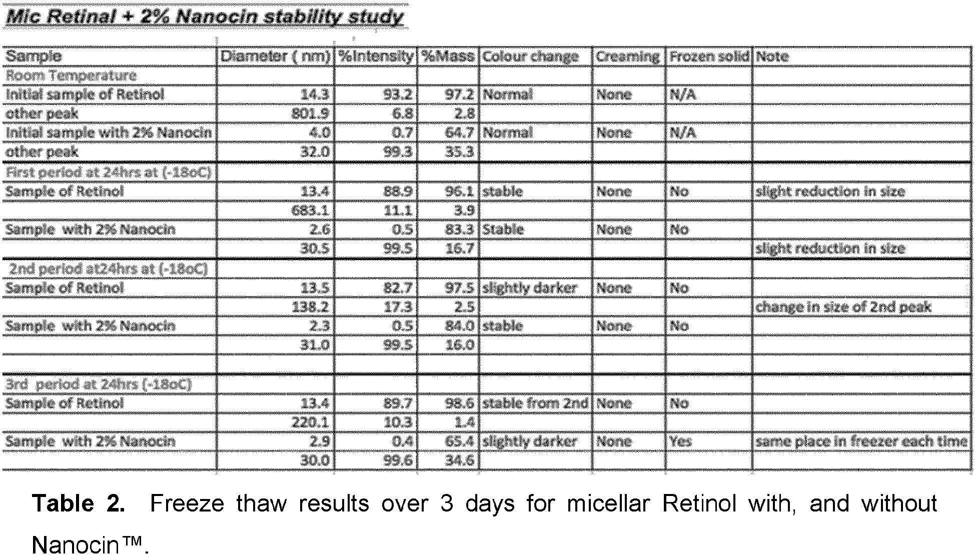

### Example 2. Experimental method and sample set-up used for NanoSight assessment of nanoparticles.

Nanocin^{™} was tested with Vitamin C in water without TPGS. Both compounds were prepared as 1 mg/ml stock solutions. Nanocin^{™} was pre-heated to 60°C for 20 minutes, vortexed then allowed to cool to room temperature.

Incubation conditions were prepared with different ratios of vitamin C:Nanocin^{™}; 1:1, 1:3, 1:5 and 1:10 with the Vitamin C concentration staying at 5 µg/ml and Nanocin^{™} starting at 5 µg/ml then increasing to 15, 25 and 50 µg/ml with the increase in ratio.

A 2 ml solution was prepared by first adding the Vitamin C to the water, vortexing, then adding the Nanocin^{™} last, followed by shearing up and down the tip 10 times. The mixture was then left at 4°C for 4 days before making a measurement on a Nanosight LM10 instrument. Readings were made under operating conditions camera level 16, detection threshold 6 and screen gain 10.

Nanocin^{™} was also tested with Retinol in either ethanol (molecular biology grade) or Propylene Glycol (PEG as shortened in the tables below or PG as a generally accepted short name). Both Nanocin^{™} and Retinol were prepared as 1 mg/ml solutions in both Propylene Glycol and ethanol. The Propylene Glycol solutions were warmed to 37°C for 30 minutes with gentle shaking to dissolve. The Retinol solutions (in Propylene Glycol or ethanol- 1 mg/ml) were stored at -20°C and wrapped in foil to prevent exposure to light.

The Retinol:Nanocin^{™} in either Propylene Glycol or ethanol were combined in a different order compared to the Vitamin C:Nanocin^{™} combination. For both the Propylene Glycol and ethanol solutions Nanocin^{™} was added to the correct volume of water first, followed by vortexing, then the Retinol was added last followed by shearing the solutions up and down 10 times. The Retinol and Nanocin^{™} solutions were pre-heated to 37°C before adding to allow dissolving to occur. Ratios of Retinol:Nanocin^{™} of 1:1, 1:3, 1:5 and 1:10 were prepared with Retinol at a final concentration of 5 µg/ml and Nanocin^{™} increasing with each ratio from 5, 15, 25 and 50 µg/ml. The samples were then left wrapped in foil at 4°C for 2 days before making a measurement using a Nanosight LM10 instrument. Readings were taken under operating conditions camera level 13, detection threshold 6 and screen gain 10. Experimental ratios are detailed in **Table 3.**

**Table 3. Experimental Samples**

| **Vitamin C: Nanocin^{™}** | **Vitamin C Volume (µl)** | **Nanocin^{™} Volume (µl)** | **Water Volume (µl)** |
|---|---|---|---|
| 1:1 | 10 | 10 | 1980 |
| 1:3 | 10 | 30 | 1960 |
| 1:5 | 10 | 50 | 1940 |
| 1:10 | 10 | 100 | 1890 |
| 5µg/ml Vitamin C | 10 | 0 | 1990 |
| 5µg/ml Nanocin^{™} | 0 | 10 | 1990 |
| 15µg/ml Nanocin^{™} | 0 | 30 | 1970 |
| 25µg/ml Nanocin^{™} | 0 | 50 | 1950 |
| 50µg/ml Nanocin^{™} | 0 | 100 | 1800 |

| **Retinol : Nanocin^{™} (in Ethanol)** | **Retinol Volume (µl)** | **Nanocin^{™} Volume (µl)** | **Water Volume (µl)** |
|---|---|---|---|
| 1:1 | 10 | 10 | 1980 |
| 1:3 | 10 | 30 | 1960 |
| 1:5 | 10 | 50 | 1940 |
| 1:10 | 10 | 100 | 1890 |
| 5µg/ml Retinol | 10 | 0 | 1990 |
| 5µg/ml Nanocin^{™} | 0 | 10 | 1990 |
| 15µg/ml Nanocin^{™} | 0 | 30 | 1970 |
| 25µg/ml Nanocin^{™} | 0 | 50 | 1950 |
| 50µg/ml Nanocin^{™} | 0 | 100 | 1800 |

| **Retinoic Acid : Nanocin^{™} (in PROPYLENE GLYCOL)** | **Retinoic Acid Volume (µl)** | **Nanocin^{™} Volume (µl)** | **Water Volume (µl)** |
|---|---|---|---|
| 1:1 | 10 | 10 | 1980 |
| 1:3 | 10 | 30 | 1960 |
| 1:5 | 10 | 50 | 1940 |
| 1:10 | 10 | 100 | 1890 |
| 5µg/ml Retinol | 10 | 0 | 1990 |
| 5µg/ml Nanocin^{™} | 0 | 10 | 1990 |
| 15µg/ml Nanocin^{™} | 0 | 30 | 1970 |
| 25µg/ml Nanocin^{™} | 0 | 50 | 1950 |
| 50µg/ml Nanocin^{™} | 0 | 100 | 1800 |

### Example 3. Formulation of particles of Retinol with Nanocin^{™}

### Objective 1: formulate particles of Retinol with Nanocin^{™}.

*Objective 1* was achieved by using alternative solvents and also by using a nanomicellar formulation with TPGS. The alternative solvents are detailed below:

### A) Water

All attempts to dissolve Retinol in water were unsuccessful except when using a micellar formulation with TPGS, as per the method described in Example 1.

### B) Ethanol

Retinol was dissolved in absolute ethanol at a concentration of 10 mg/ml with Nanocin^{™} added at the concentrations indicated in tables 4-9 below. Two techniques were used to evaluate the creation of nanoparticles; direct visual observation using fluorescence microscopy and non-imaging detection via dynamic light scattering (DLS) using a Malvern Zetasizer instrument. Observation using a fluorescence microscope (excitation: 335 nm, emission: 458 nm) revealed small, illuminated dots, which quickly faded (not shown). This establishes the formation and presence of Retinol and Nanocin^{™} particles. Retinol shows fluorescence with emission wavelength 458 nm. Using the dynamic light scattering (DLS) technique (Malvern Zetasizer) allowed the actual size of the particles of Retinol dissolved in absolute ethanol with different concentrations of Nanocin^{™} to be determined. DLS is a well-established technique used in submicron particle size analysis. 10 mg/ml Retinol (in absolute ethanol) alone had an average size of 396.5 nm and this increased upon addition of various concentrations of Nanocin^{™}, which indicates the creation of particles (see **Figure 2** and **Table 4**).

**Table 4. The average size of particles of Retinol dissolved in absolute ethanol with individual concentrations of Nanocin^{™} determined by DLS**

| Sample | Average size [d.nm] | Standard Deviation |
|---|---|---|
| 10 mg/ml Retinol (in absolute ethanol): 0 mg/ml Nanocin^{™} | 396.5 | 7.99 |
| 10 mg/ml Retinol (in absolute ethanol): 10 mg/ml Nanocin^{™} | 888.7 | 72.72 |
| 10 mg/ml Retinol (in absolute ethanol): 100 mg/ml Nanocin^{™} | 1437.0 | 68.97 |

### C) Propylene Glycol

Retinol was dissolved in propylene glycol at a concentration of 10 mg/ml. The presence of particles of Retinol dissolved in propylene glycol with Nanocin^{™} (seen as fluorescent particles) was determined by observation under a fluorescence microscope.

Observation under a fluorescence microscope (excitation: 335 nm, emission: 458 nm) revealed the presence of small, illuminated dots, which quickly faded (not shown). This indicated formation of Retinol and Nanocin^{™} particles. Retinol shows fluorescence with an emission wavelength of 458 nm.

Unfortunately, the precise size of particles of Retinol dissolved in propylene glycol (PEG) with different concentrations of Nanocin^{™} could not be determined reproducibly using DLS (Malvern Zetasizer) due to a high polydispersity of the particle suspension.

### D) 5% Micellar Solution of Retinol with TPGS and Nanocin

5% micellar solution of Retinol was also used with different concentrations of Nanocin^{™}. As described previously, visual observation (fluorescence microscopy) was used to determine if particles had been created. Again, DLS (Malvern Zetasizer) was used to determine the size distribution of particles.

Observation under fluorescence microscope (excitation: 335 nm, emission: 458 nm) did not show the presence of particles. However, this does not preclude the creation of particles since the particles may be too small to be seen by this method (see below).

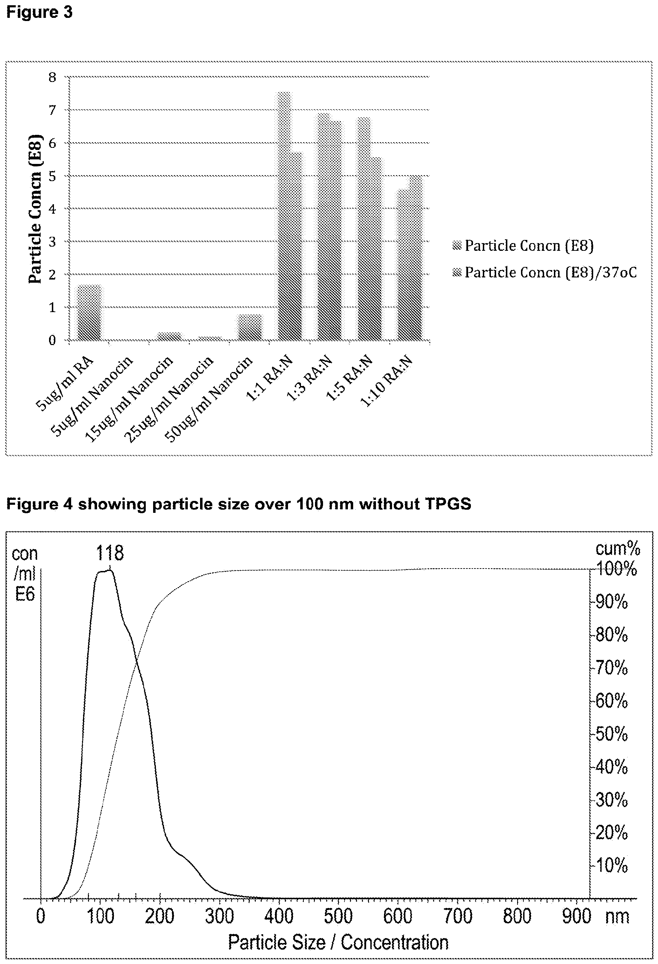

DLS detection using the Malvern Zetasizer instrument clearly revealed the formation of nanoparticles. The 5% micellar solution of Retinol alone had an average nanoparticle size of 13.08 nm which increased upon addition of various concentrations of Nanocin^{™}, (see **Figure 4** and **Table 5**).

**Table 5. The average size of nanoparticles of 5% micellar solution of Retinol with individual concentrations of Nanocin^{™}.**

| Sample | Average size [d.nm] | Standard Deviation |
|---|---|---|
| 5% micellar solution of Retinol: 0% Nanocin^{™} | 13.08 | 0.12 |
| 5% micellar solution of Retinol: 2% Nanocin^{™} | 14.12 | 0.07 |
| 5% micellar solution of Retinol: 5% Nanocin^{™} | 16.61 | 0.16 |
| 5% micellar solution of Retinol: 10% Nanocin^{™} | 19.57 | 0.28 |

### Example 4. Determination of Retinol and Nanocin^{™} particle stability

*Objective 2* determine stability and capability for delivering Vitamins of formulated particles.

*Objective 2* was achieved. Stable particles of Retinol and Nanocin^{™} have been generated.

The stability of formulated particles was confirmed by following the absorbance as a function of storage time.

### A) Water

Retinol. No research was undertaken on *Objective 2* because Retinol is insoluble in water.

### B) Ethanol

Unlike the retinoic acid control sample in Propylene Glycol (PEG), the control Retinol in ethanol sample did not show a high level of particles, however the samples were measured unheated and at 37°C. **Figure 3** shows a comparison of particle concentration on the incubation of Retinol and Nanocin^{™} (in ethanol) after 2 days at 4°C with, or without warming to 37°C before reading. The particle sizes for the Retinol:Nanocin^{™} combination ranged from 104 nm -130 nm (warmed to 37°C) or 103 nm -113 nm not warmed. The distribution profiles of particle sizes with the differing ratios were very similar (see below). **Figure 4** shows the particle size distribution for 1:3 Retinol: Nanocin^{™} in ethanol.

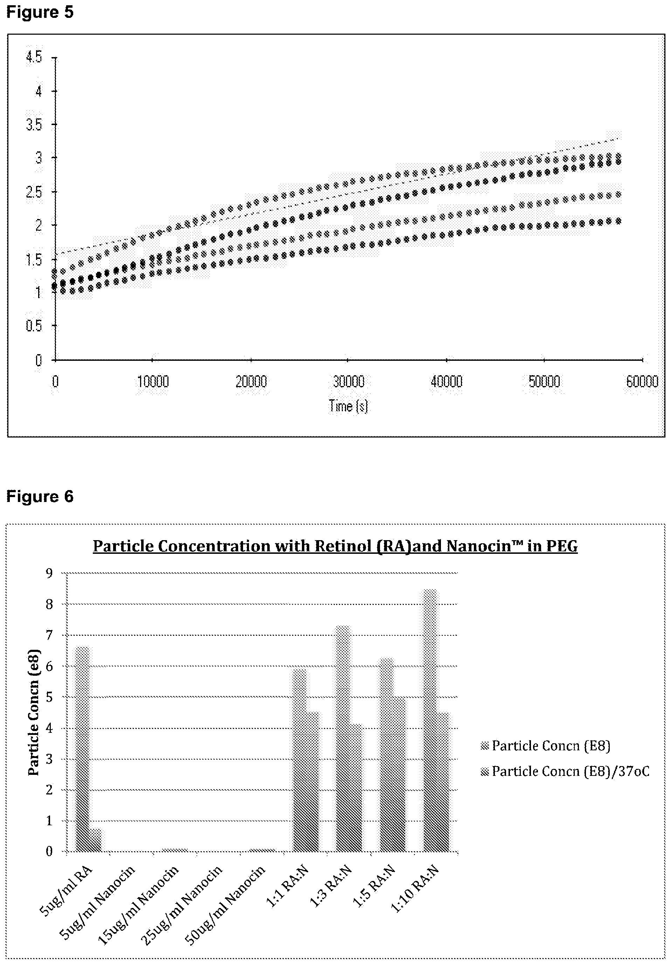

The solutions of 10 mg/ml Retinol in absolute ethanol with different concentrations of Nanocin^{™} were stored for 24 hours at room temperature in the dark. The solution was also protected against degradation by placing in amber glass vials and storing in the dark. To assess the stability of the particles, measurement of absorbance with wavelength of 405 nm was performed after the initial preparation at 0 hour storage and after 24 hours' storage. Kinetics absorbance measurements (with wavelength of 405 nm) of particles of Retinol dissolved in absolute ethanol with different concentrations of Nanocin^{™} (10 mg/ml Retinol alone, with 10 mg/ml, 50 mg/ml and 100 mg/ml Nanocin^{™}) was also measured during 24 hours' storage time see (**Table 6** and **Figure 5**).

**Table 6. The absorbance of Retinol dissolved in absolute ethanol with individual concentrations of Nanocin^{™} at time zero (0 h) and after 24 h storage.**

| Sample | A₄₀₅ | A₄₀₅ |
|---|---|---|
| | 0 h | 24 h |
| 10 mg/ml Retinol (in absolute ethanol) : 0 mg/ml Nanocin^{™} | 1.79 | 2.66 |
| 10 mg/ml Retinol (in absolute ethanol) : 10 mg/ml Nanocin^{™} | 1.58 | 2.73 |
| 10 mg/ml Retinol (in absolute ethanol) : 50 mg/ml Nanocin^{™} | 1.62 | 3.36 |
| 10 mg/ml Retinol (in absolute ethanol) : 100 mg/ml Nanocin^{™} | 1.72 | 2.86 |

In summary, after 2 days incubation at 4°C with Retinol and Nanocin^{™} in ethanol, all the ratios tested gave large increases in particle concentrations compared with control samples. With warming, the concentrations decreased but still remained greater than the control values with the 1:3 ratio giving the largest concentration of nanoparticles formed. Particles were stable in solution/ suspension and modest chemical stabilisation was observed.

### C) Propylene Glycol

After 2 days incubation at 4°C, initial results showed that the Retinol control (in 0.5% Propylene Glycol) gave a high concentration of particles, so the samples were then warmed to 37°C for 30 minutes with gentle shaking to dissolve any particles and the results were compared as unheated and heated at 37°C, as shown in **Figure 6****.**

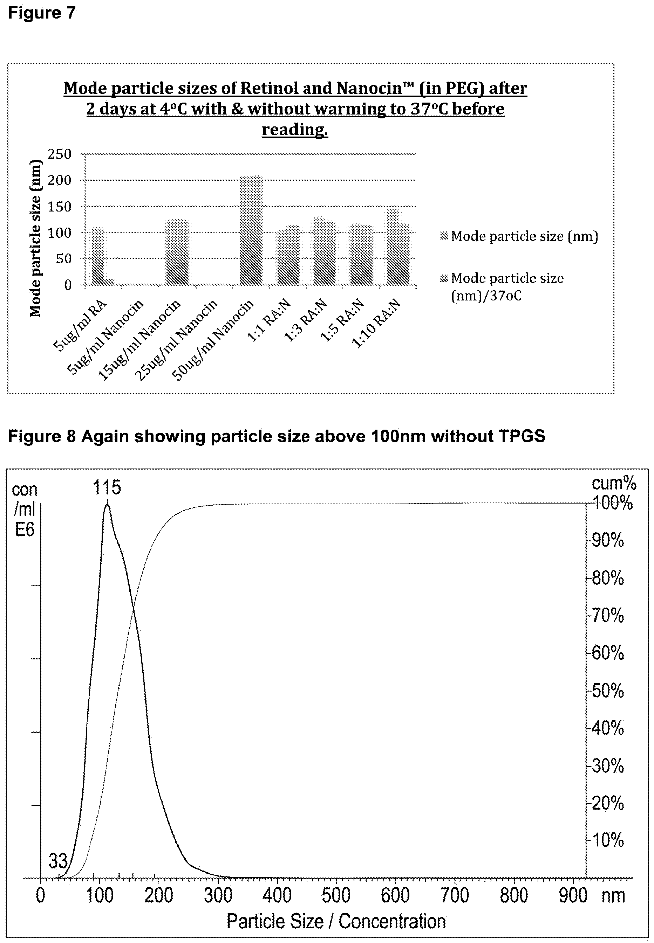

There was a considerable decrease in particle concentration in the Retinol control after warming; all the Nanocin^{™} controls had very few or no particles present. All combinations of retinoic acid: Nanocin^{™} showed a significant increase in particle concentrations compared with the controls, which dropped slightly after warming. The modal particle sizes of Retinol and Nanocin^{™} (in Propylene glycol (PEG or PG)) was measured after 2 days at 4°C with and without warming to 37°C before reading (**Figure 7**). Typically the average particle size for the warmed Retinol:Nanocin^{™} samples without TPGS ranged from 115 nm-121 nm with a consistent size distribution width. **Figure 8** shows the particle size distribution for 1:5 Retinol: Nanocin^{™} in Propylene Glycol (PEG or PG) without TPGS.

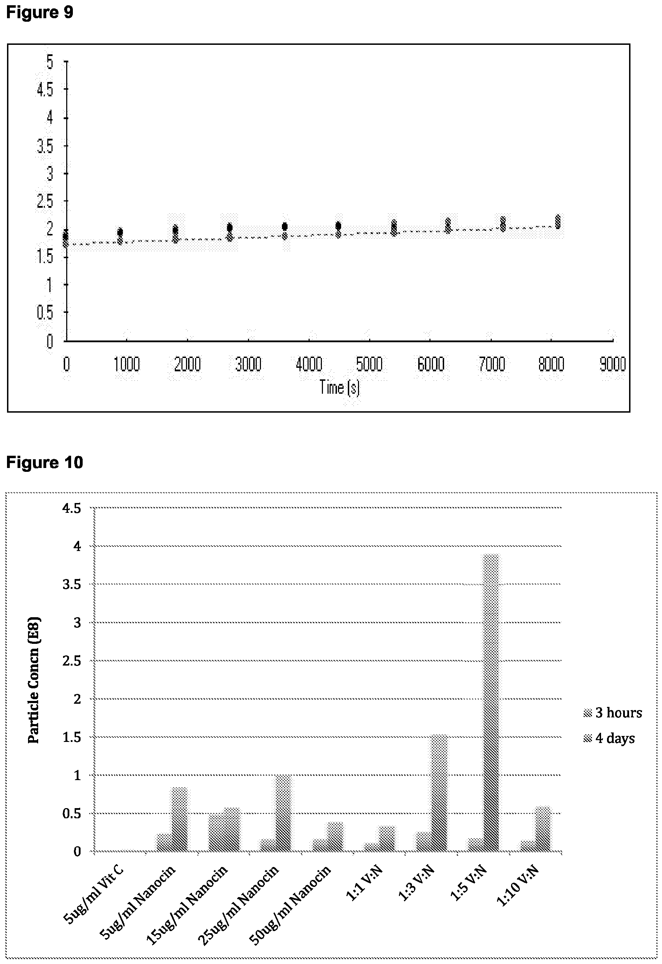

Solutions of 10 mg/ml Retinol in Propylene Glycol (PEG) with different concentrations of Nanocin^{™} were stored for 24 hours at room temperature. The solutions were also protected against potential degradation by storing in amber glass vials in the dark. To assess the stability of the nanoparticles, measurement of absorbance with wavelength of 405 nm was performed at 0 h storage and after 24 h storage. Kinetics absorbance of particles of Retinol dissolved in Propylene Glycol with different concentrations of Nanocin^{™} (10 mg/ml Retinol alone, with 10 mg/ml, 50 mg/ml and 100 mg/ml Nanocin^{™}) was also measured (measurement of absorbance with wavelength of 405 nm) during 24 h storage time (see **Table 7** and **Figure 9**).

**Table 7. The absorbance of Retinol dissolved in Propylene Glycol with individual concentrations of Nanocin^{™} at 0 h and after 24 h storage.**

| Sample | A₄₀₅ | A₄₀₅ |
|---|---|---|
| | 0 h | 24 h |
| 10 mg/ml Retinol (propylene glycol) : 0 mg/ml Nanocin^{™} | 2.37 | 3.31 |
| 10 mg/ml Retinol (propylene glycol) : 10 mg/ml Nanocin^{™} | 2.47 | 2.37 |
| 10 mg/ml Retinol (propylene glycol) : 50 mg/ml Nanocin^{™} | 2.66 | 2.58 |
| 10 mg/ml Retinol (propylene glycol) : 100 mg/ml Nanocin^{™} | 2.61 | 2.63 |

In summary, after 2 days incubation at 4°C with Retinol and Nanocin^{™} in Propylene Glycol (PEG) resulted in considerable increases in particle concentrations after combining, compared with the controls. All the ratio combinations showed similar levels of particles concentrations with 1:5 (Retinol: Nanocin^{™}) giving the greatest concentration level. The variation in particles sizes were very similar between all the ratios and ranged from 115 nm -121 nm. Particles were stable in solution/suspension and modest chemical stabilisation was observed under some conditions.

### D) 5% Micellar Solution of Retinol with TPGS (Example 1 method).

5% micellar solutions of Retinol with different concentrations (0 %, 2%, 5%, 10%) of Nanocin^{™} was stored for 5 days at room temperature in the dark. To assess the stability of particles measurement of absorbance with a wavelength of 405 nm was performed after initial preparation at 0 days storage and after 5 days storage (see **Table 8).**

**Table 8. The absorbance of particles of 5% micellar solution of Retinol with individual concentrations of Nanocin^{™} at time zero (0 days) and after 5 days' storage.**

| Sample | A₄₀₅ | A₄₀₅ |
|---|---|---|
| | 0 day | 5 day |
| 5% micellar solution of Retinol: 0% Nanocin^{™} | 4.11 | 4.18 |
| 5% micellar solution of Retinol : 2% Nanocin^{™} | 4.05 | 4.40 |
| 5% micellar solution of Retinol: 5% Nanocin^{™} | 4.04 | 4.51 |
| 5% micellar solution of Retinol : 10% Nanocin^{™} | 4.14 | 4.19 |

To assess particle stability, solutions of particles were centrifuged on the fifth storage day and measurement of absorbance at 405 nm was performed before and after centrifugation (see **Table 9**).

**Table 9. The absorbance of particles of 5% micellar solution of Retinol with individual concentrations of Nanocin^{™} on storage day 5 before and after centrifugation.**

| Sample | A₄₀₅ | A₄₀₅ |
|---|---|---|
| | Before centrifugation | After centrifugation |
| 5% micellar solution of Retinol: 0% Nanocin^{™} | 4.18 | 4.50 |
| 5% micellar solution of Retinol : 2% Nanocin^{™} | 4.40 | 4.47 |
| 5% micellar solution of Retinol: 5% Nanocin^{™} | 4.51 | 4.38 |
| 5% micellar solution of Retinol : 10% Nanocin^{™} | 4.19 | 4.34 |

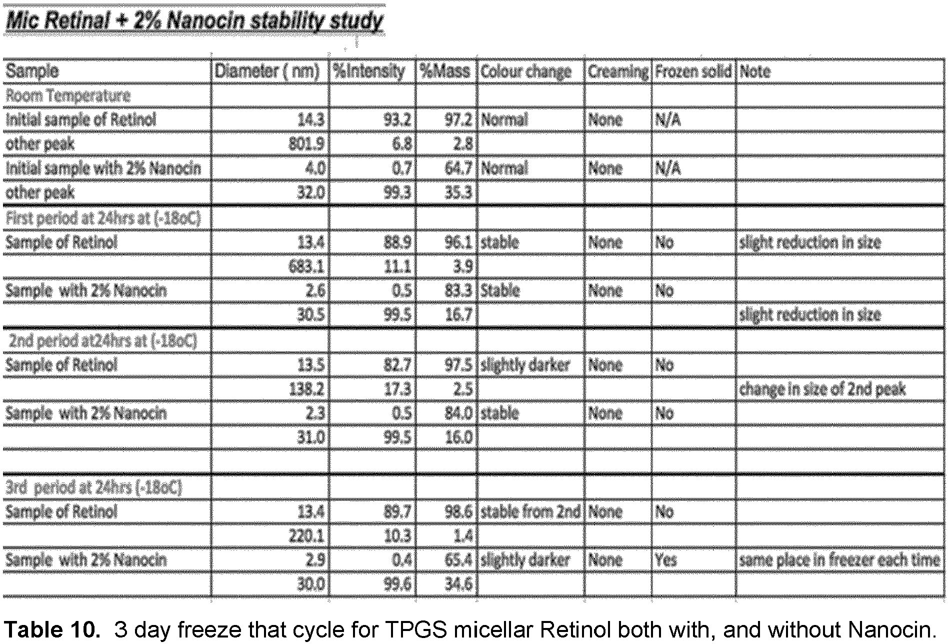

### Example 5. Formulation of particles of Vitamin C with Nanocin^{™}

***Objective 1:*** formulate nanoparticles of Vitamin C with Nanocin^{™}, without TPGS
***Objective 2:*** determine stability and capability for delivering Vitamins of formulated nanoparticles.

Nanocin^{™} was incubated with Vitamin C (in water) (with no oil or TPGS) and particle numbers determined after 3 hours and also 4 days at 4°C. After 3 hours incubation there was little difference in particle numbers so the incubations were left at 4°C for 4 days (**Figure 10**).

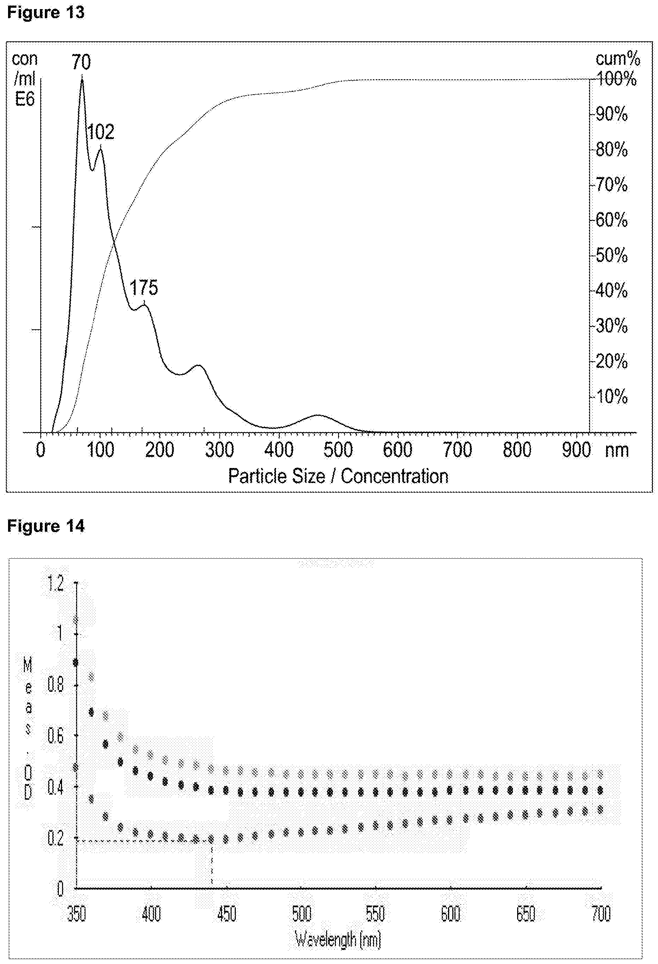

No micelles were formed but a 'nest' of buffered PHMB in water, the loading is not high. There was a single example (Figure 13) of a particle less than 100nm, 70nm, the shape of the graph is broad which may mean not distinct particles as such, but again the loading is not high and there are no micelles.

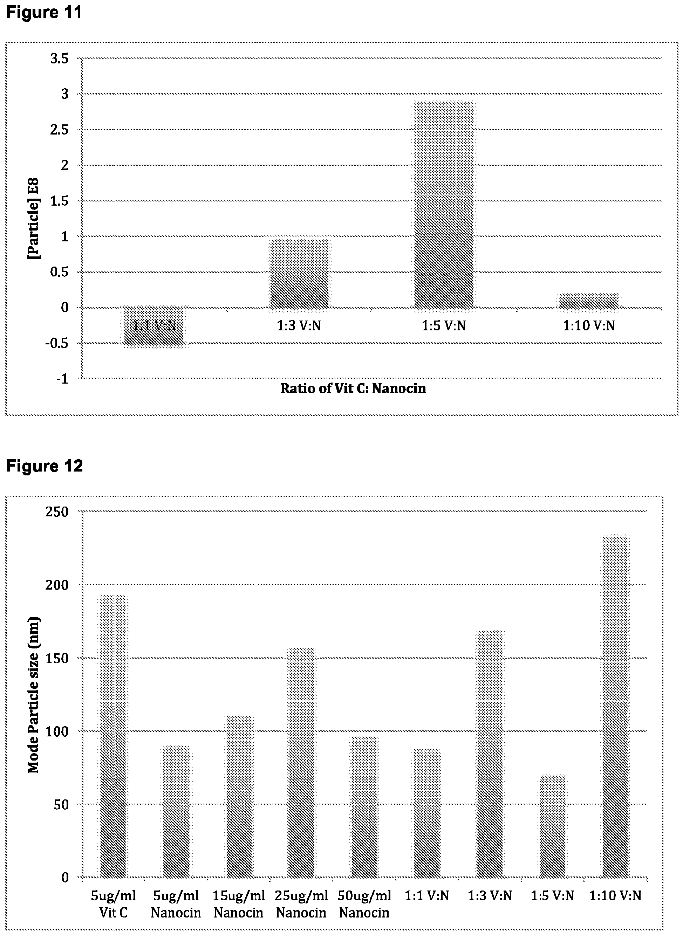

After the control values were subtracted from the combination of Vitamin C:Nanocin^{™} values, it was shown that no nanoparticles were produced at a ratio of 1:1 but were created at ratios of 1:3 and 1:5 and fewer at a 1:10 ratio. The 1:5 ratio of Vitamin C:Nanocin^{™} produced both the highest particle concentration (**Figure 11**), and the smallest particle sizes (**Figure 12**). Particle size distribution for the 1:5 Vitamin C:Nanocin^{™} mixture is shown in **Figure 13****.**

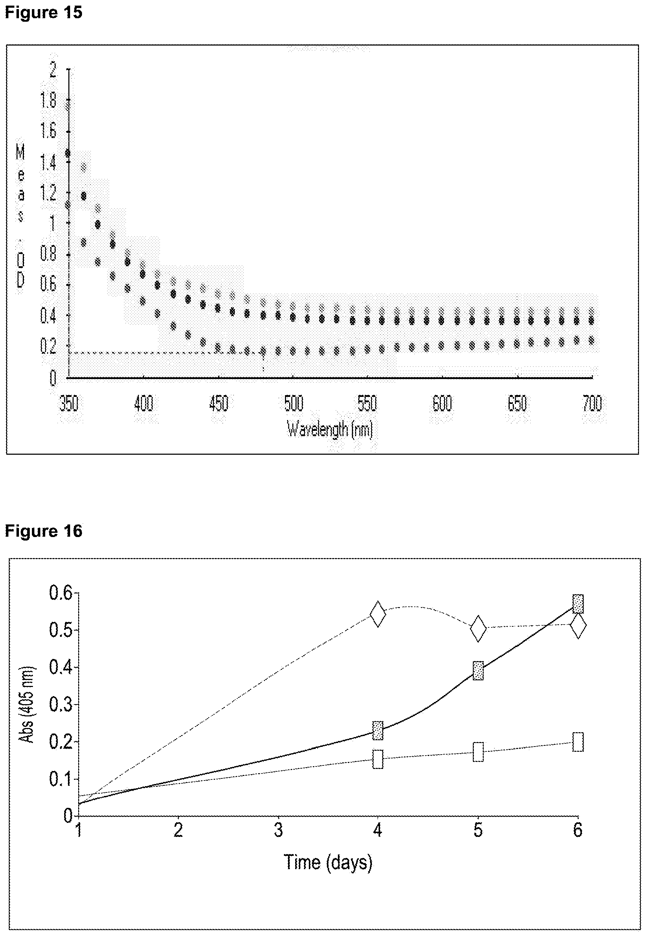

The solutions of Vitamin C in water with different concentrations of Nanocin^{™} were stored at 50°C for 6 days. Measurement of absorbance with different wavelengths (350 nm - 700 nm) was performed every 24 hours until 6 days of incubation (see **Figure 14** and **Figure 15**)**.** Measurement of the kinetics absorbance at a wavelength of 405 nm was performed at 24 hours storage and after 6 days storage (see **Figure 16**).

In summary, after 4 days incubation at 4°C with Vitamin C; the ratio giving the greatest number of nanoparticles was 1:5 (Vitamin C:Nanocin^{™}), with a modal particle size of 70 nm (**Figure 12**). The 1:5 ratio resulted in approximately 2.5 times more particles than the 1:3 ratio and 7 times more than the 1:10 ratio. The 1:1 ratio did not produce any particles. Modest chemical stabilisation was observed.

### Example 6. Formulation of micellar nanoparticles of Retinol and Ascorbyl Palmitate with TPGS and no Nanocin^{™} or PHMB

For certain applications it may be desirable to incorporate more than one bio-active agent into the micellar composition.

Accordingly, the micellar composition may comprise Retinol and Ascorbyl Palmitate. Where the micellar composition comprises Retinol and Ascorbyl Palmitate, the amount of Retinol will be in the range 30 to 99.9 of that of Ascorbyl Palmitate.

Method was performed as in Example 1.
a) Retinol and Ascorbyl Palmitate were dissolved in an organic solvent (ethanol) to provide a hydrophobic phase;
b) hydrophobic phase was added into aqueous TPGS; and
c) a portion of the organic solvent was removed by (a combination of heating/vacuum?).

It was found that replacing Retinol completely for Ascorbyl Palmitate in the micellar method described in Example 1 gives a cloudy precipitate (no micelles formed).

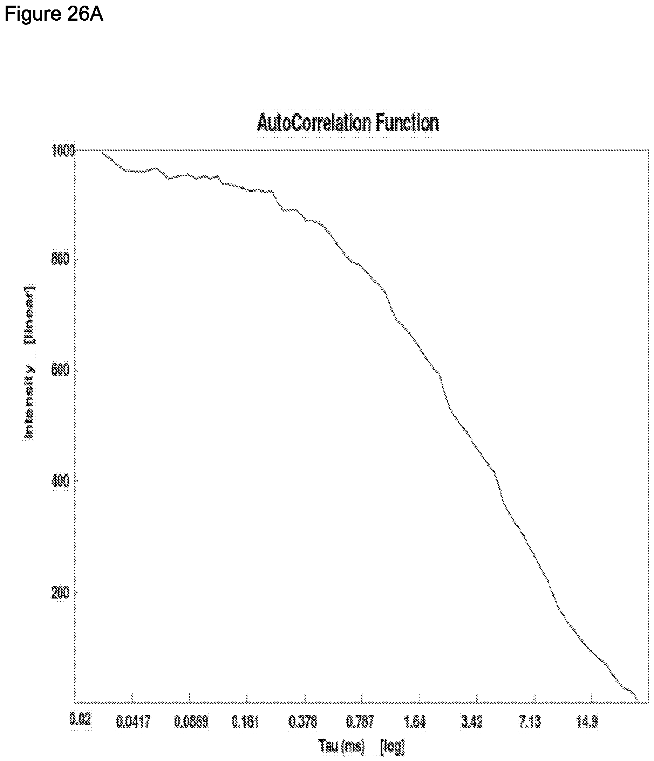

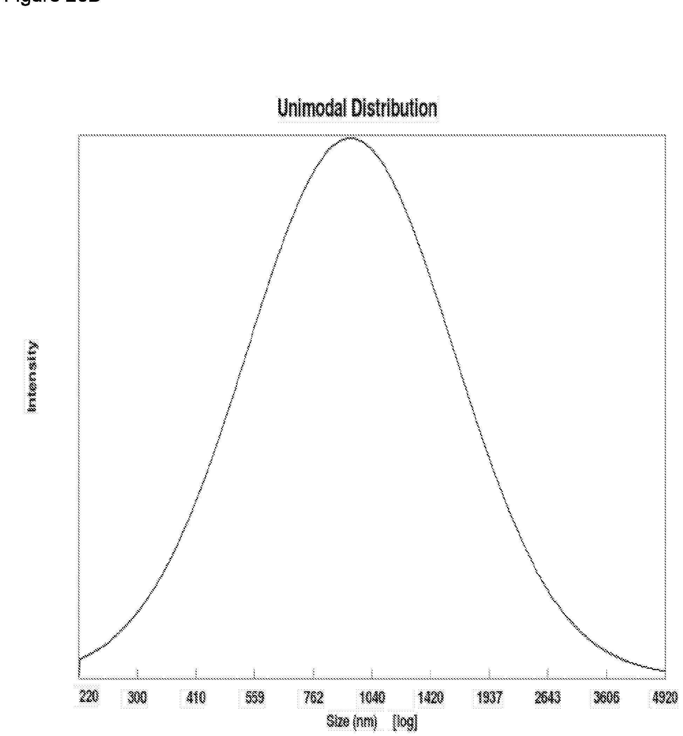

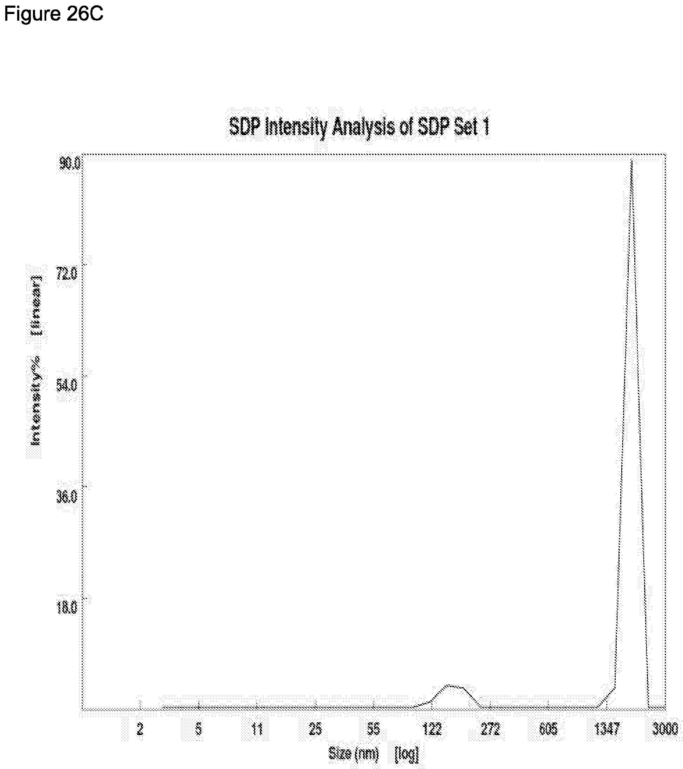

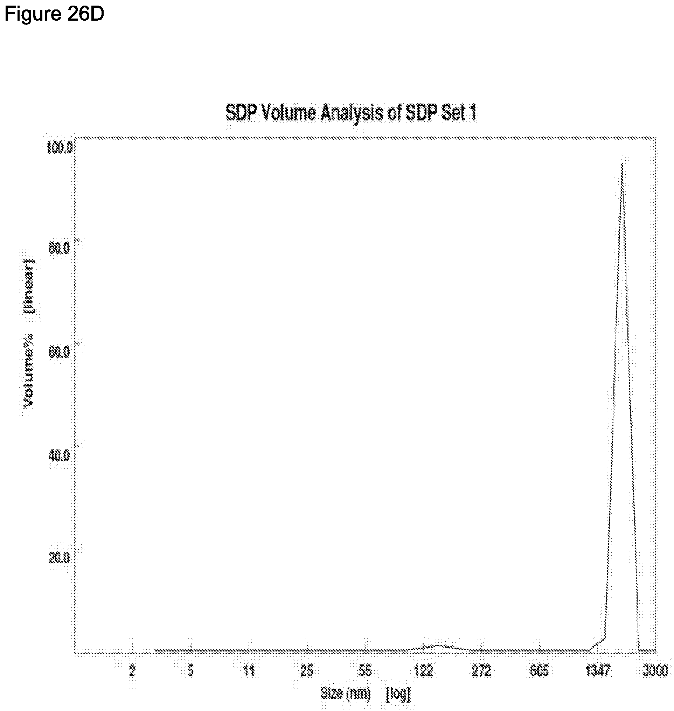

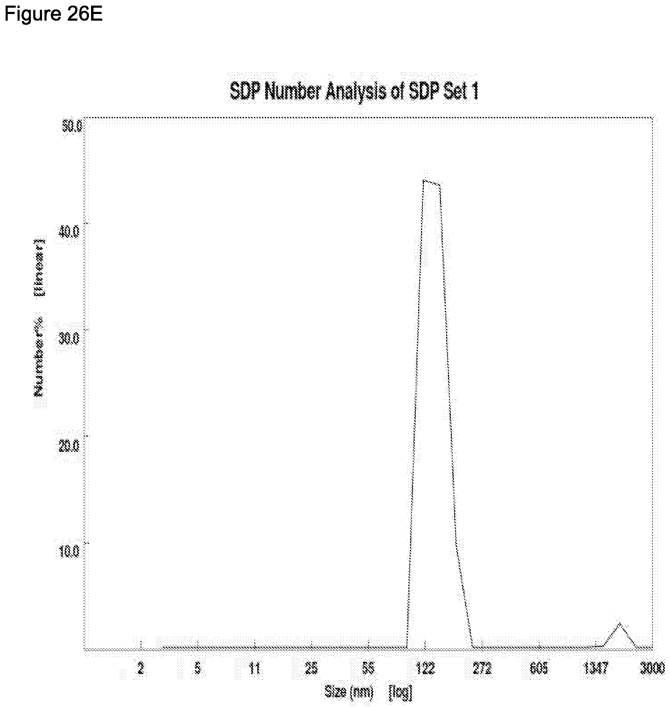

At a ratio of 20:80 Retinol:Ascorbyl Palmitate larger material considerably above nanoparticle size (100 nm) (DLS was measured prior to ethanol removal) and a cloudy gel when ethanol was removed (see **Figure 26**).

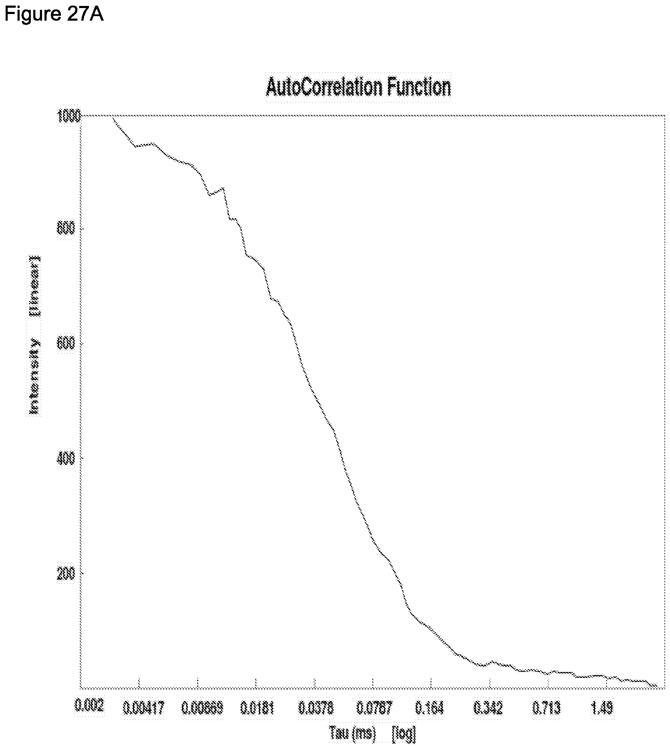

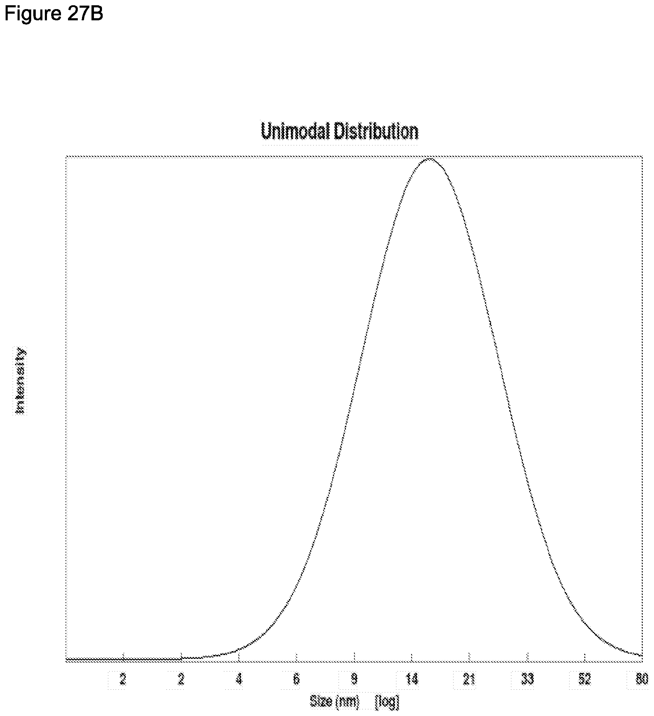

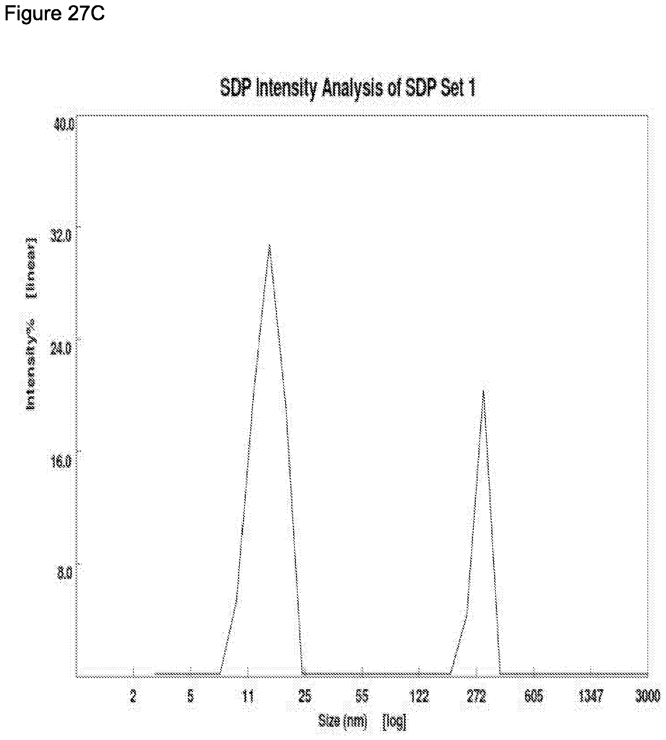

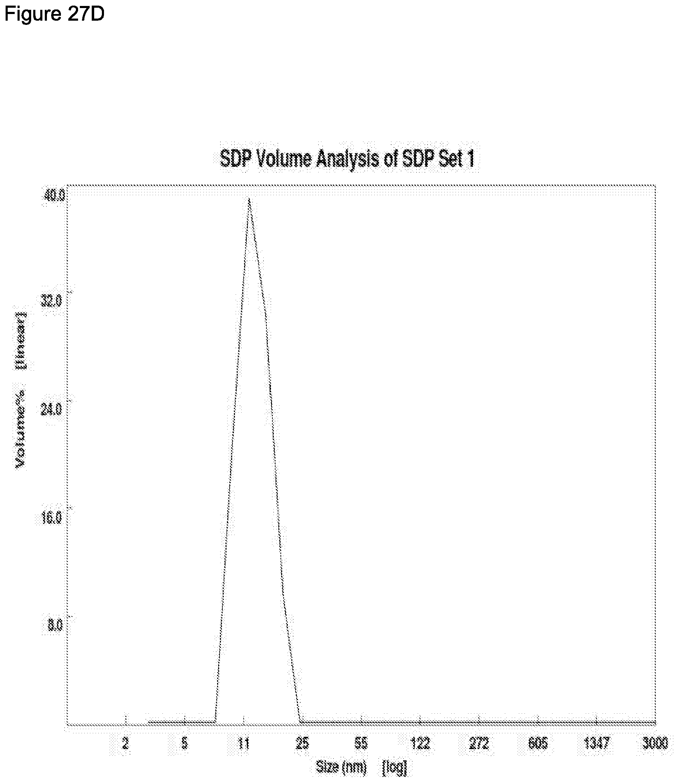

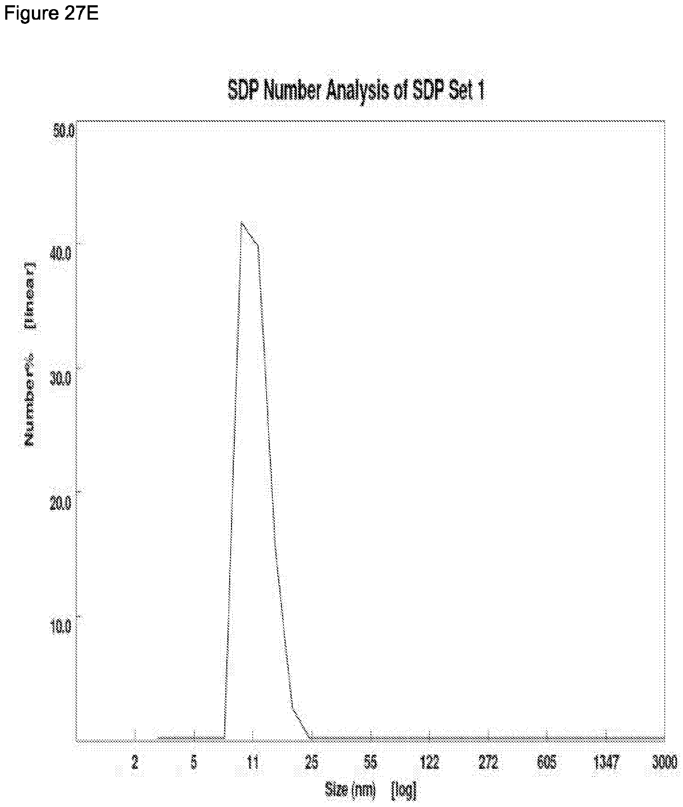

At a ratio of 50:50 Retinol:Ascorbyl Palmitate small micelles were measured (**Figure 27**).

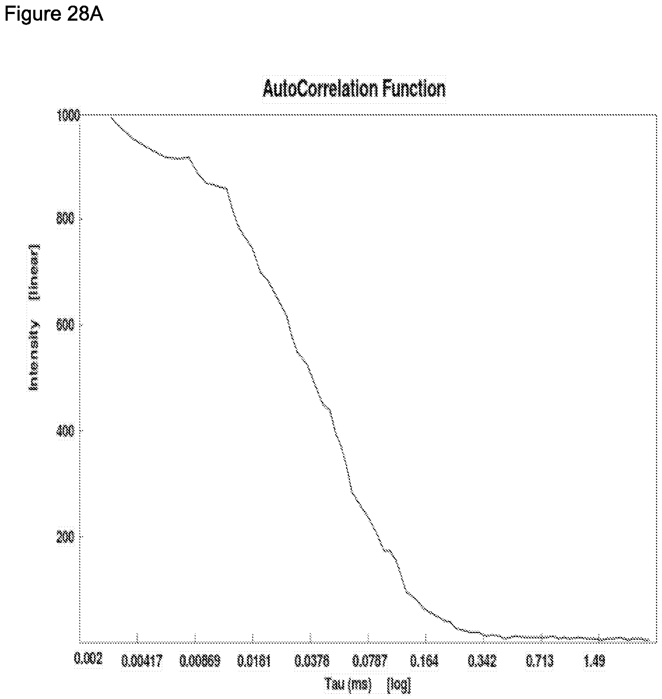

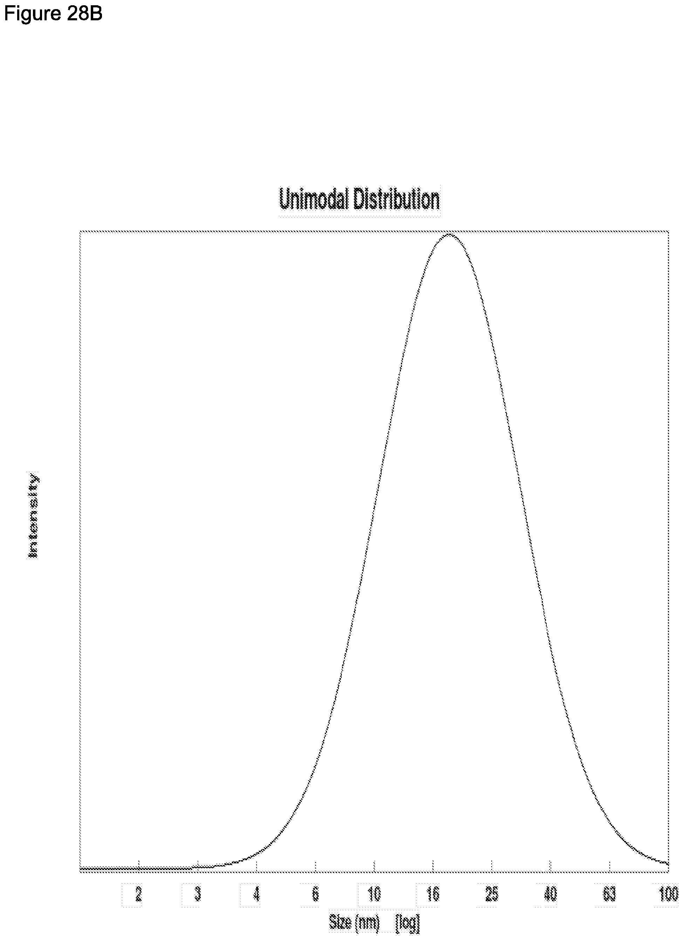

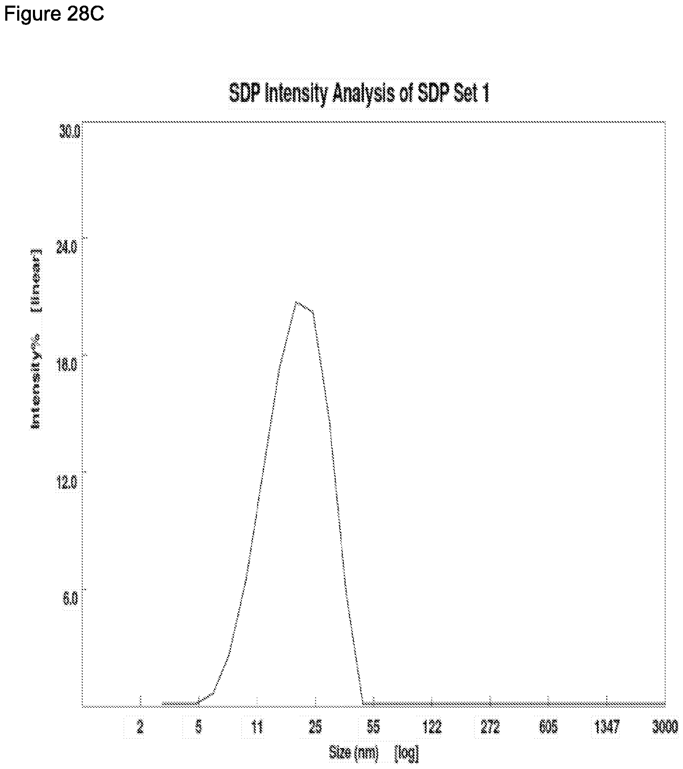

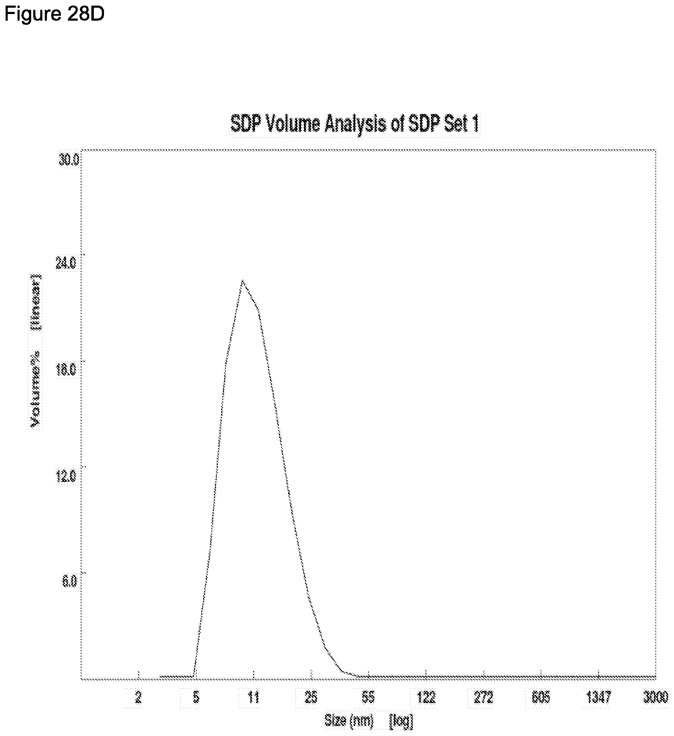

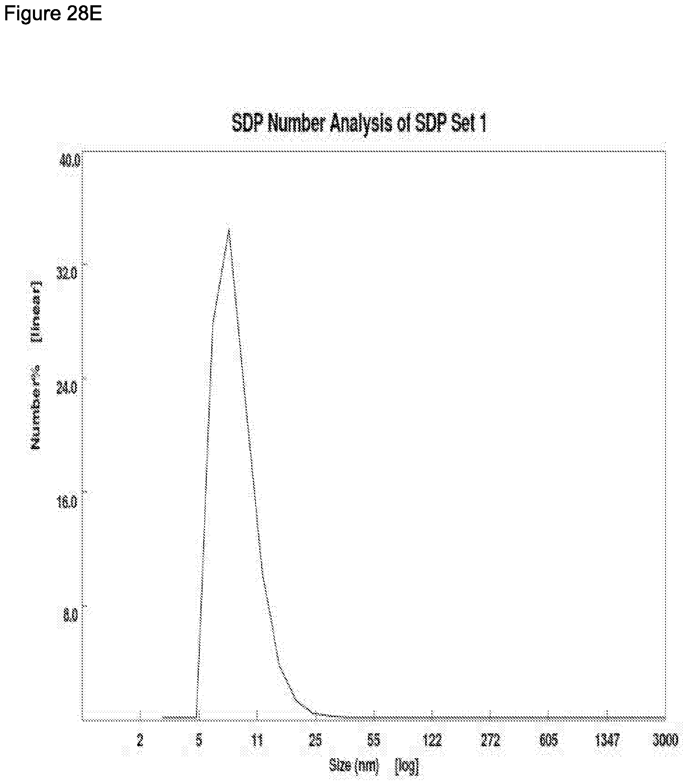

At a ratio of 90:10 Retinol:Ascorbyl Palmitate small micelles were measured (see **Figure 28**).

### Example 7. Formulation of micellar nanoparticles of Tocopherol and Tocotrienol with TPGS and without Nanocin

The method was carried out as described in Example 1 replacing retinol with tocotrienol.

The micelle formulation was used for forming micelles with Tocopherol, Tocotrienol and soy oil. The results from DLS measurements highlighted below show nano-size measurements for Tocopherol, Tocotrienol. The results for soy oil are larger.

Both the Tocopherol and Tocotrienol liquids were clear when heated to 40°C but on cooling formed a slight precipitate which was removed by filtration and then both liquid materials were clear. Heating the soy oil to 40°C resulted in a heavy precipitate that was filtered out but the liquid remained cloudy at all temperatures (no micelles).

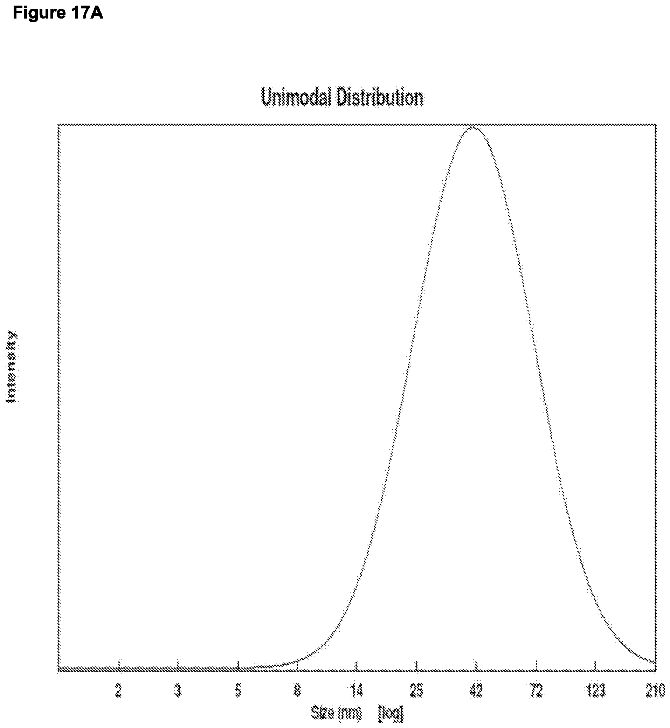

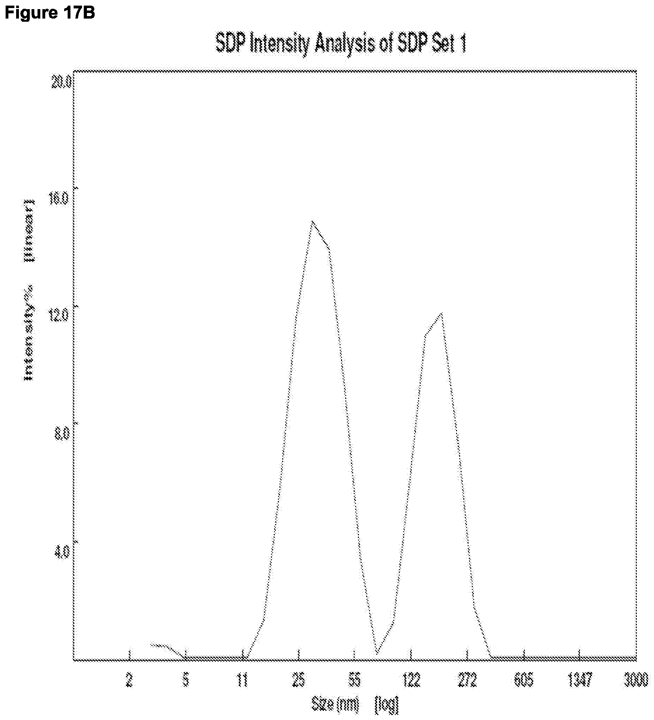

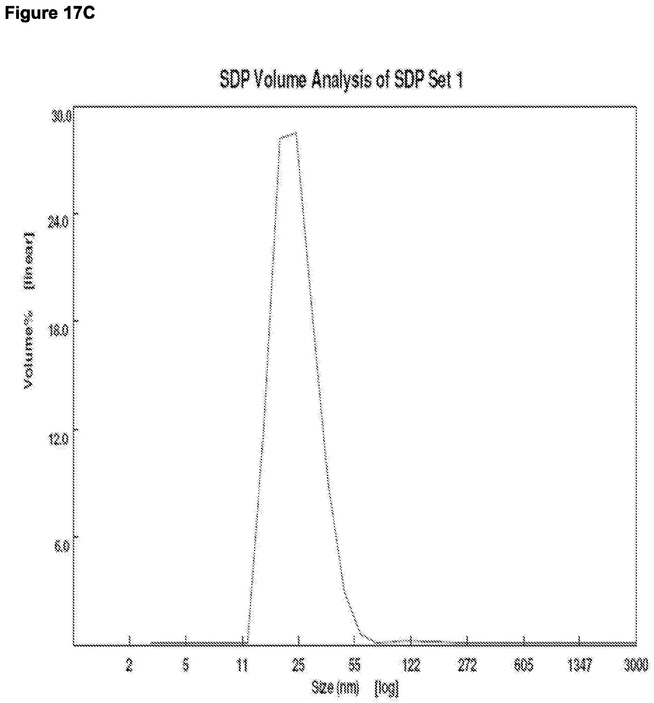

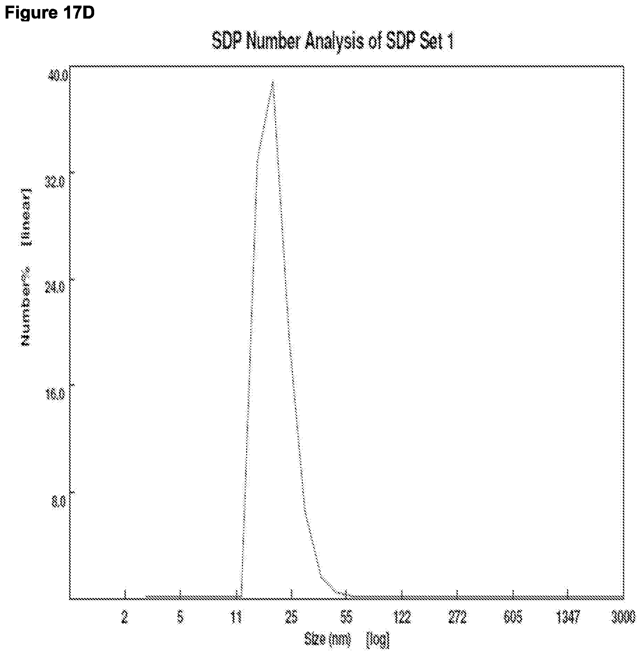

Results for micelles of Tocopherol with TPGS using method of Example 7 are detailed in **Figure 17.** Figure 17 panel B shows a peak over 100nm which is not seen in Figure 17 D, so this is a small amount of larger particle that can be dismissed for Tocopherol.

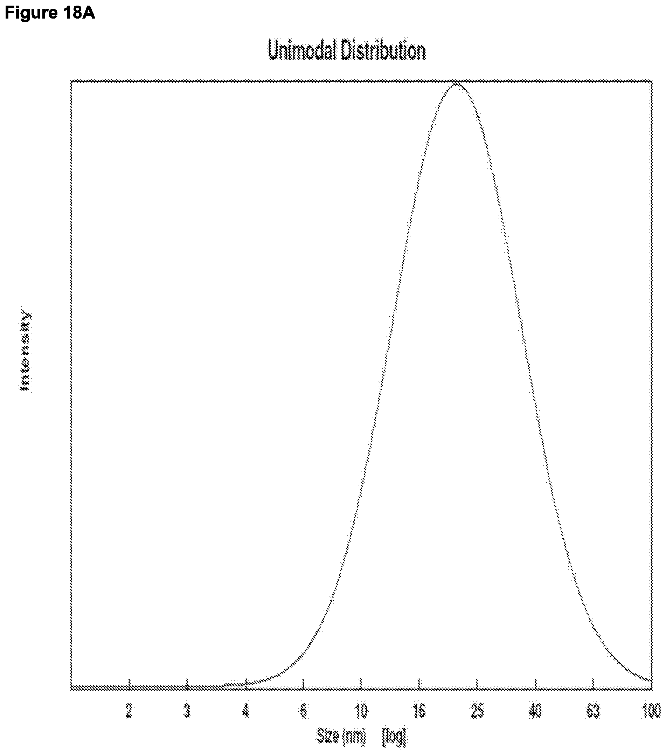

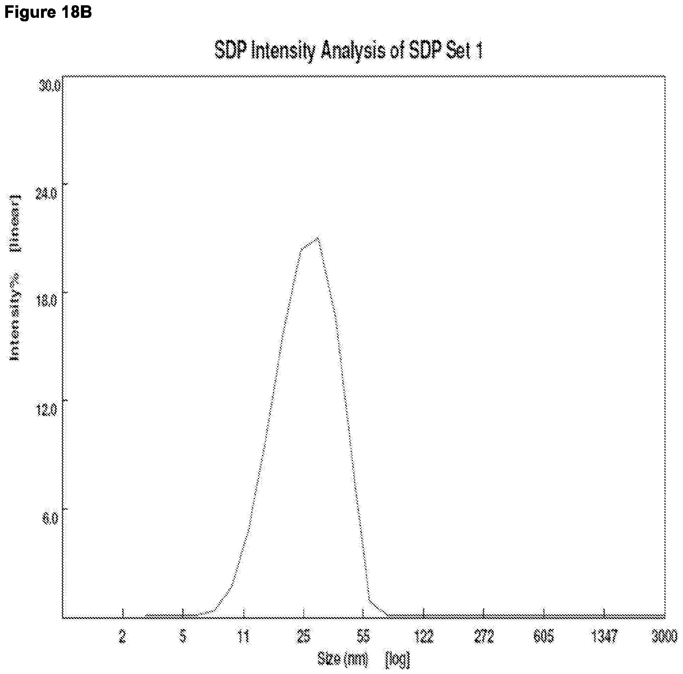

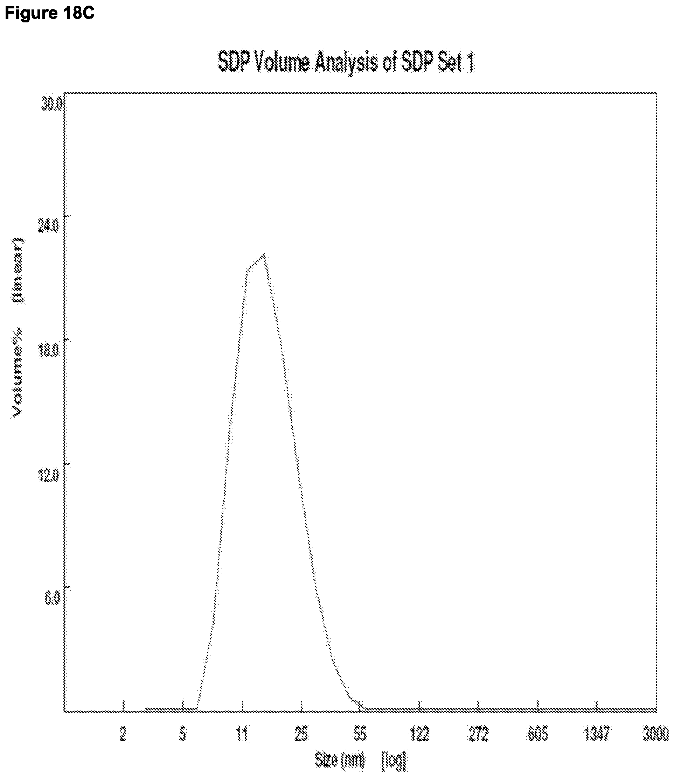

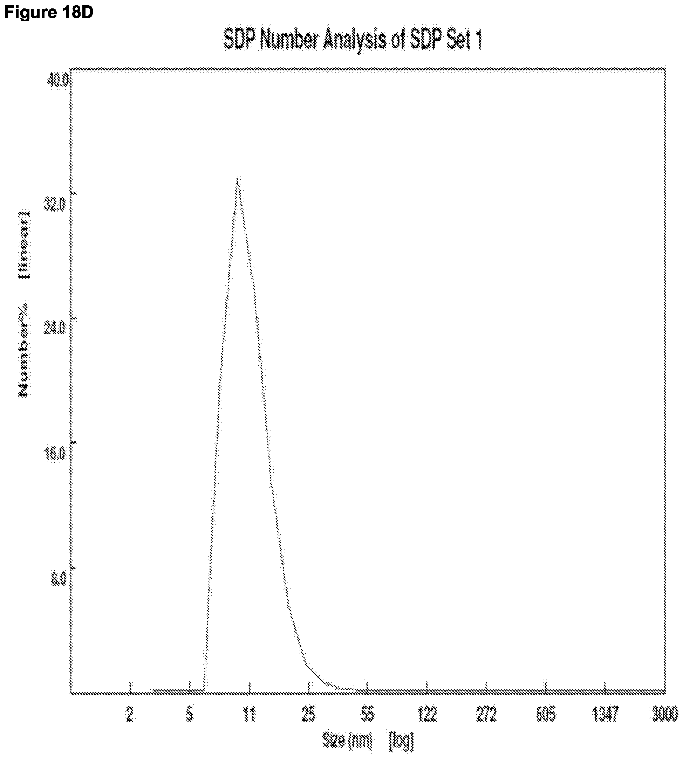

### Results for micelles of Tocotrienol according to Example 7 are detailed below and in Figure 18.

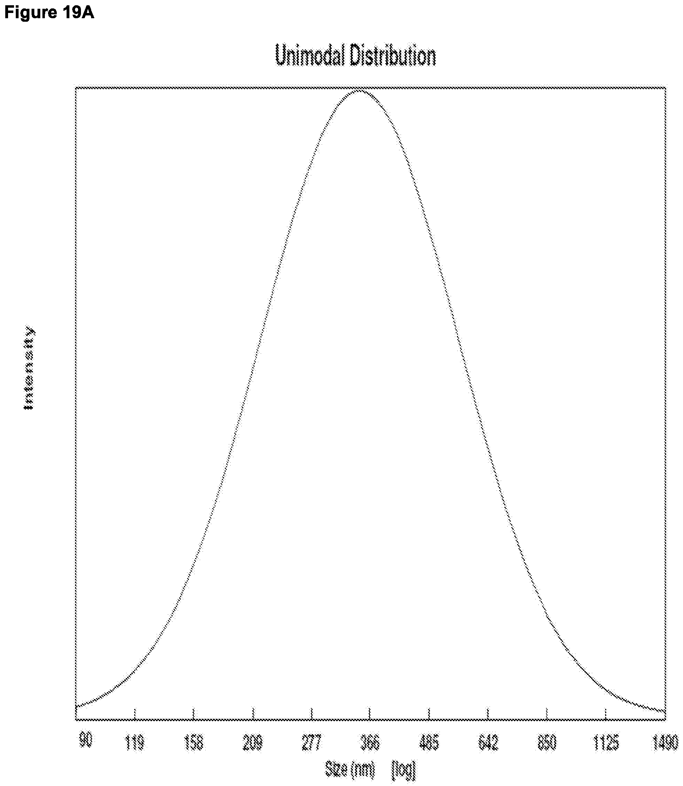

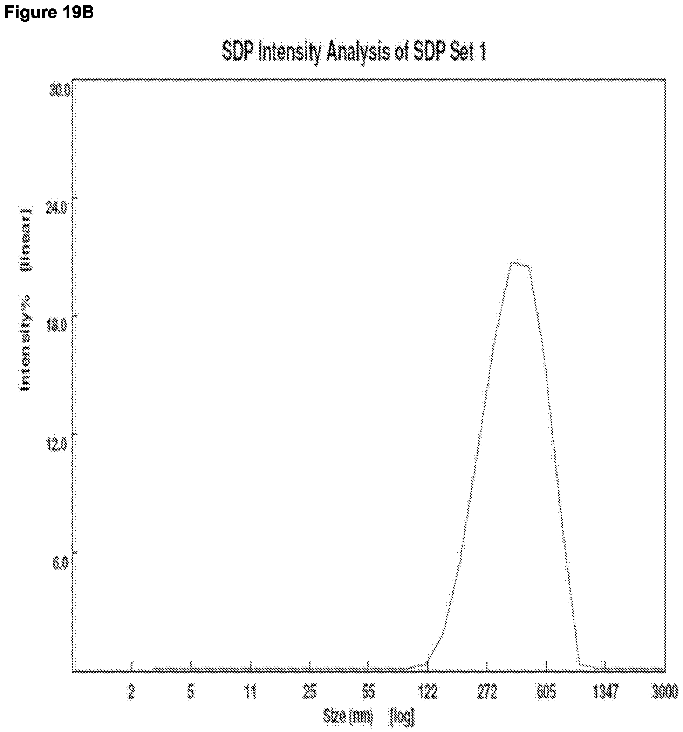

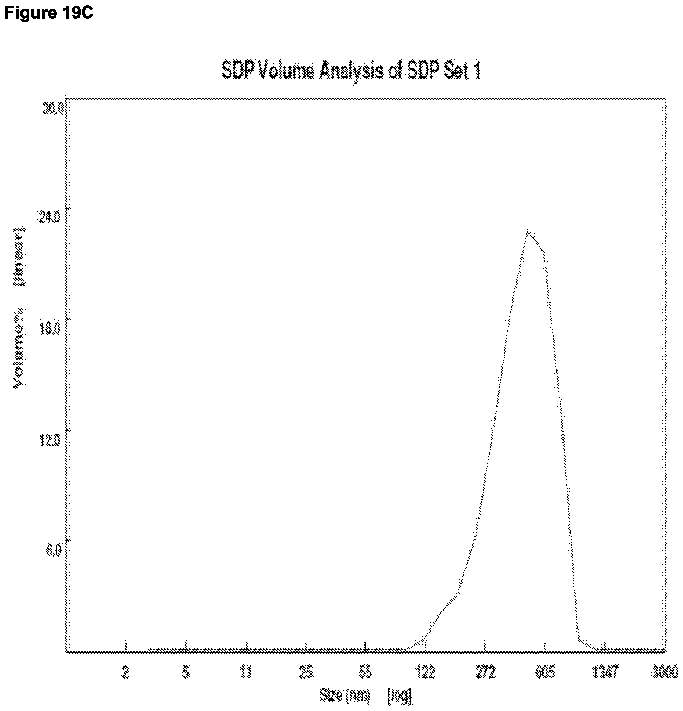

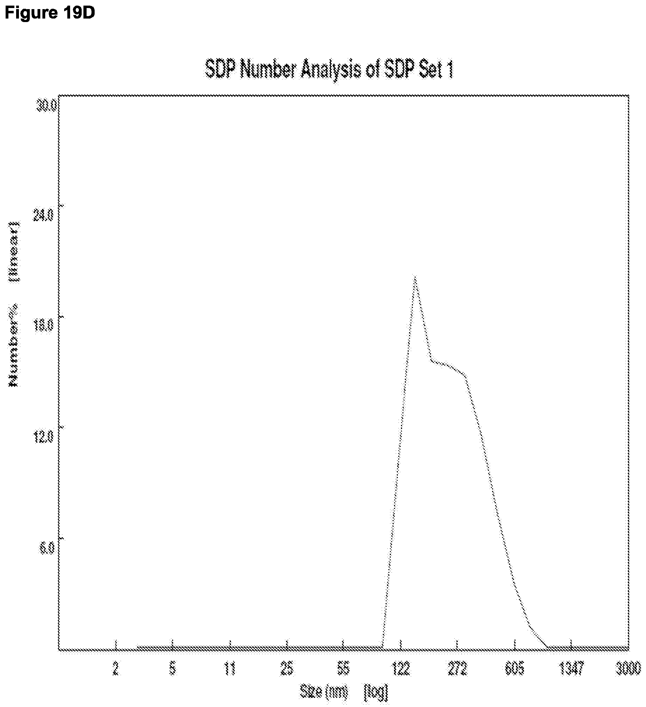

Results for Soy Oil are detailed below and in **Figure 19****.**

Throughout DLS measurements were made using a Beckman Coulter sub-micron particle analyser instrument at 20 deg C.

### Example 8: Reverse Micelle Formation

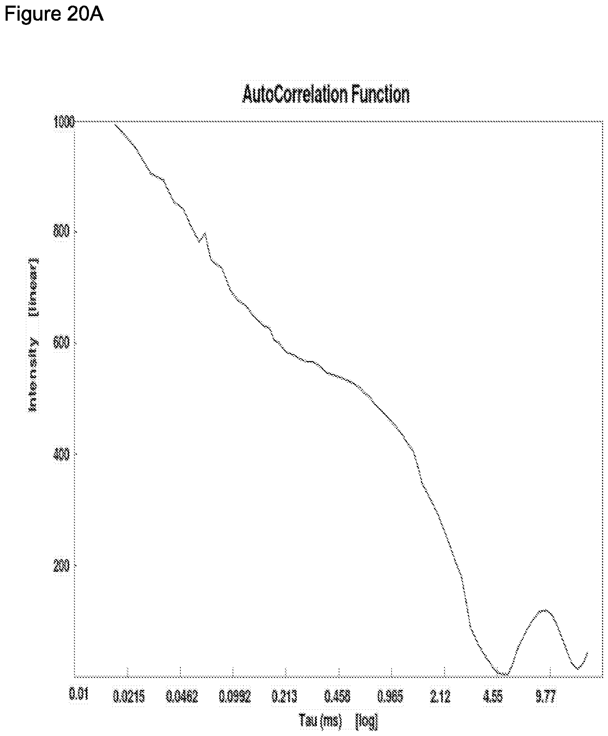

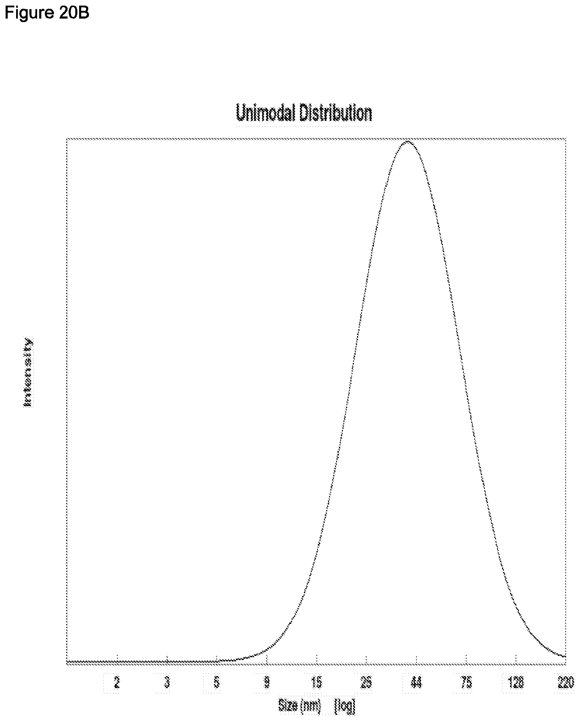

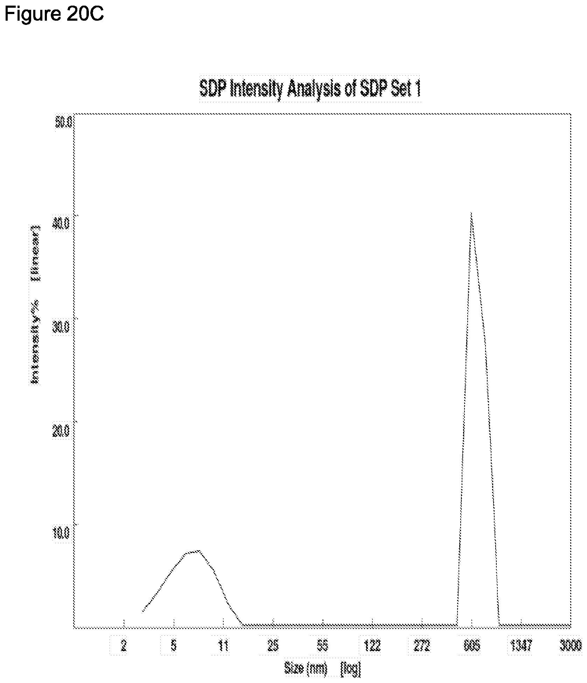

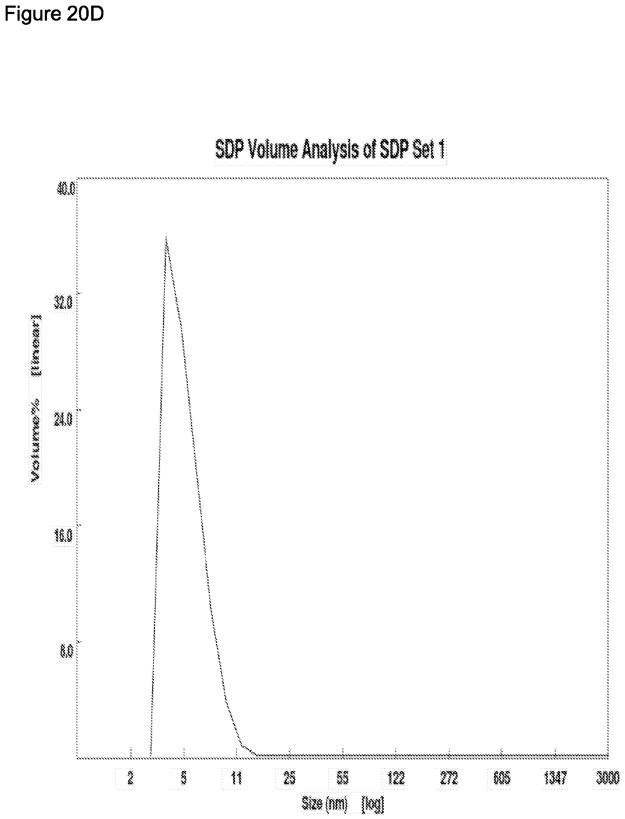

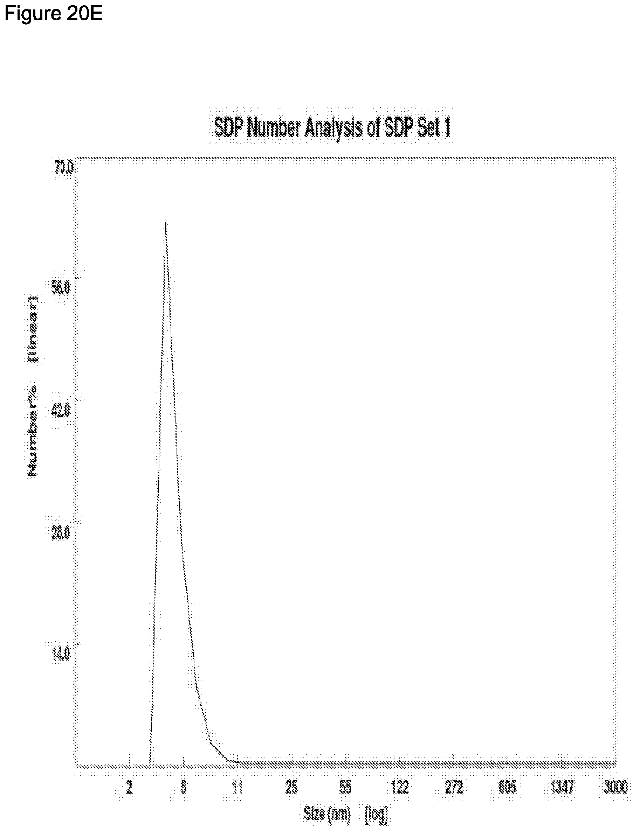

The method for Figures 20-21 is as follows: retinol was dissolved with ethanol and then 10% w/w TPGS added and both warmed, the ethanol was then driven off using warming or sonication or both forming a first solution. 5% Nanocin as a second solution was then added to the first solution to form reverse micelles. Figure 20 shows the first solution formed with 10% TPGS in retinol.

**Figure 20** shows 10% TPGS in Retinol (lipophilic) with Nanocin^{™} added in to form reverse micelle.

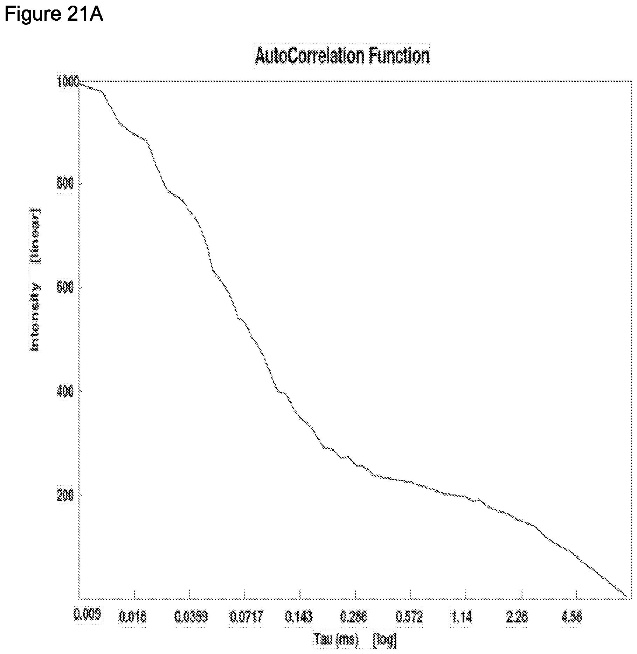

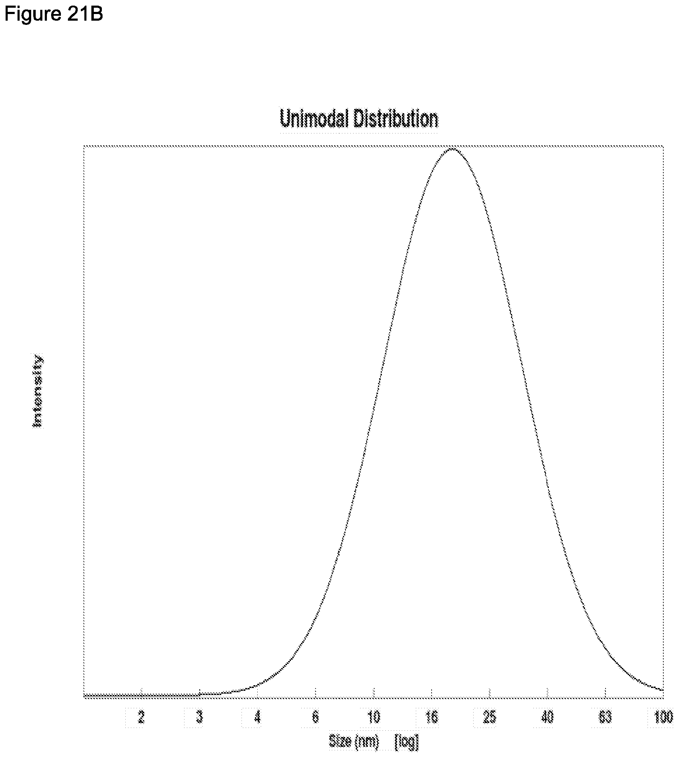

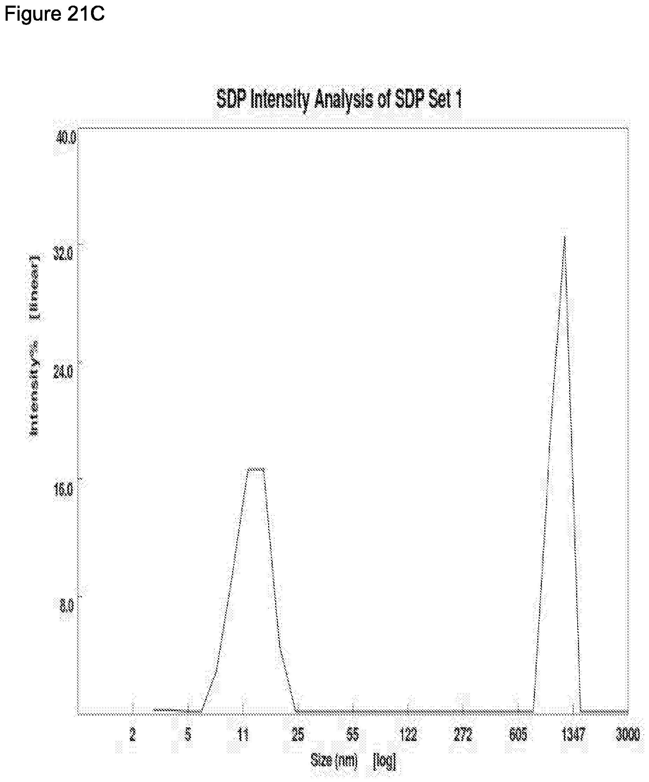

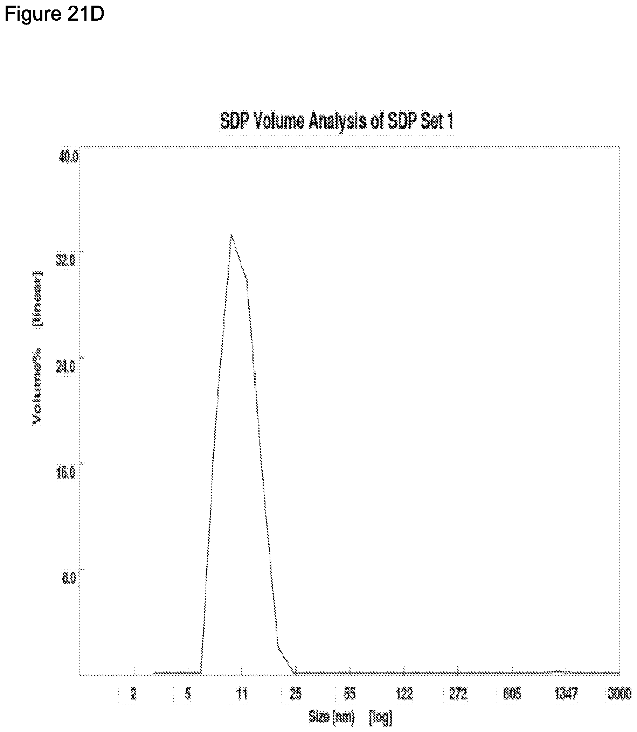

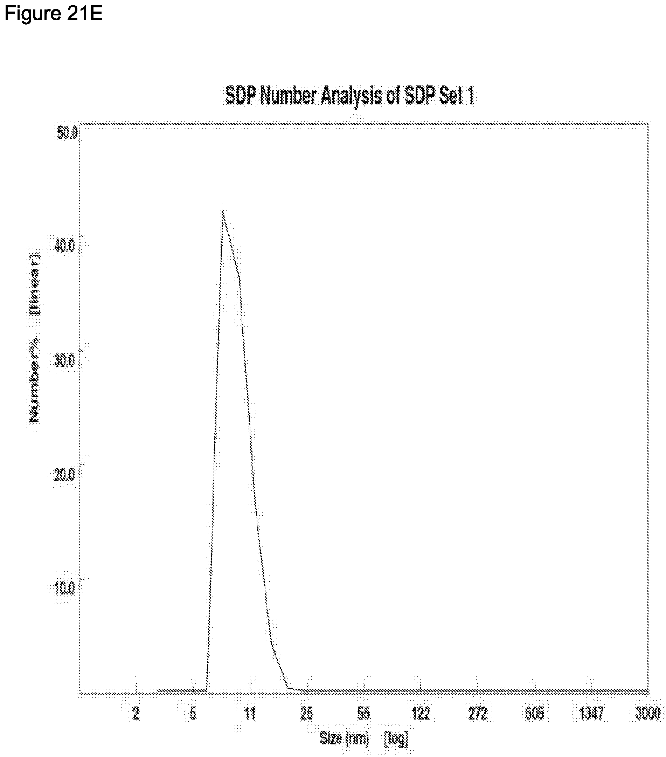

**Figure 21** shows 10% TPGS in Retinol only reverse micelle.

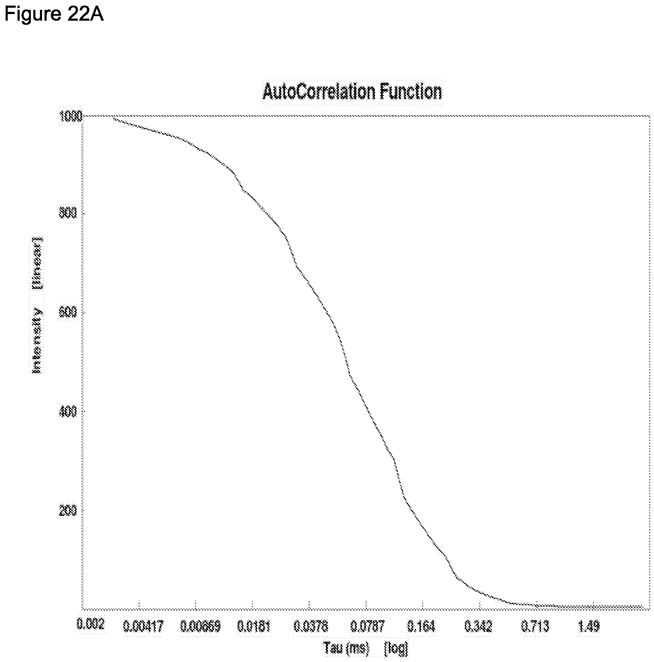

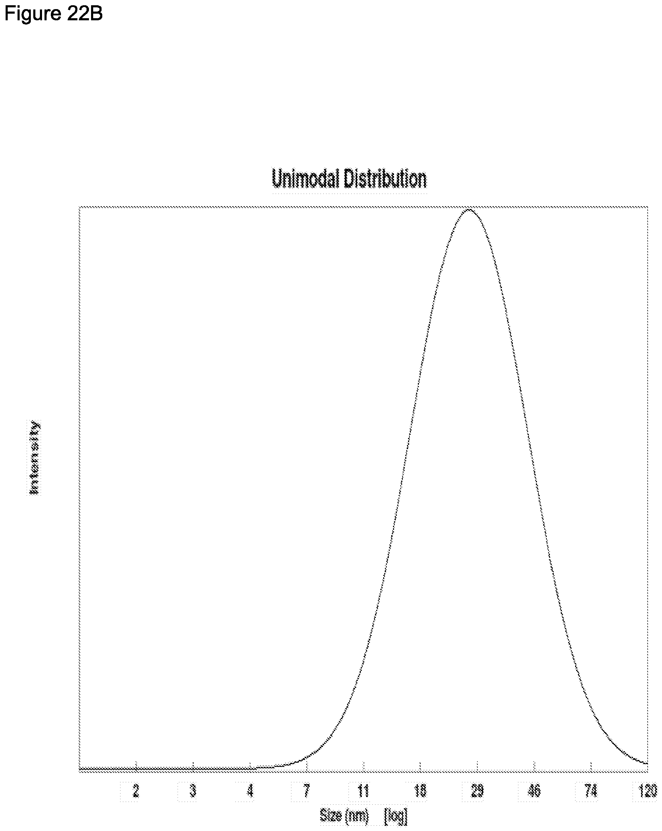

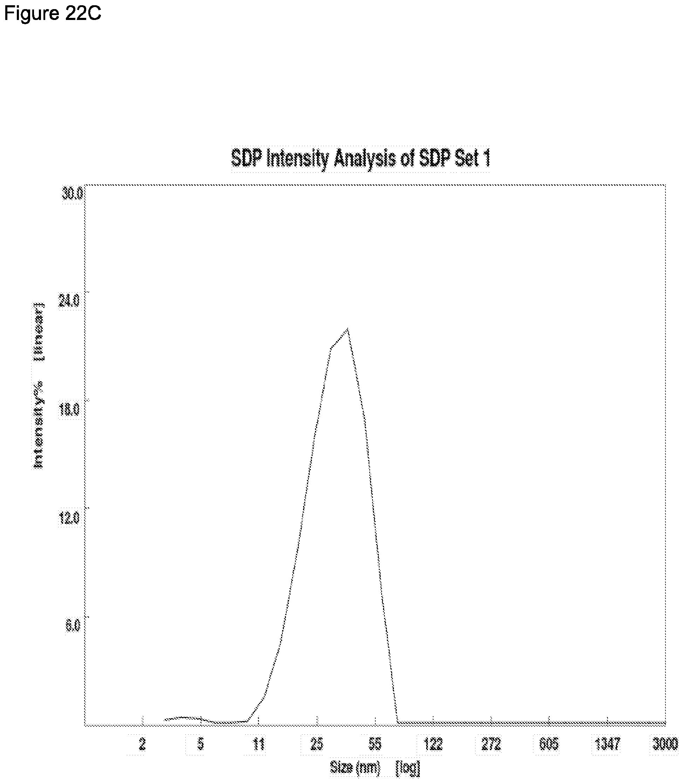

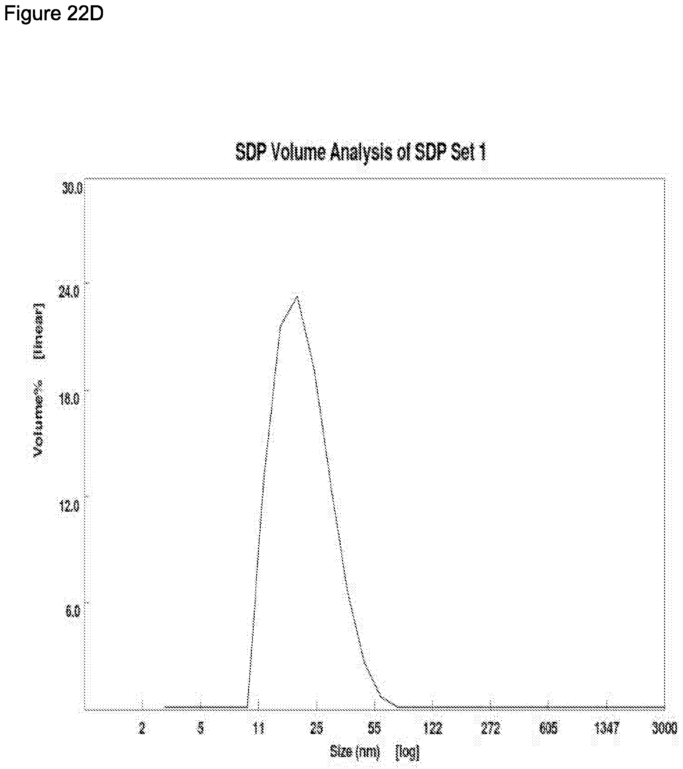

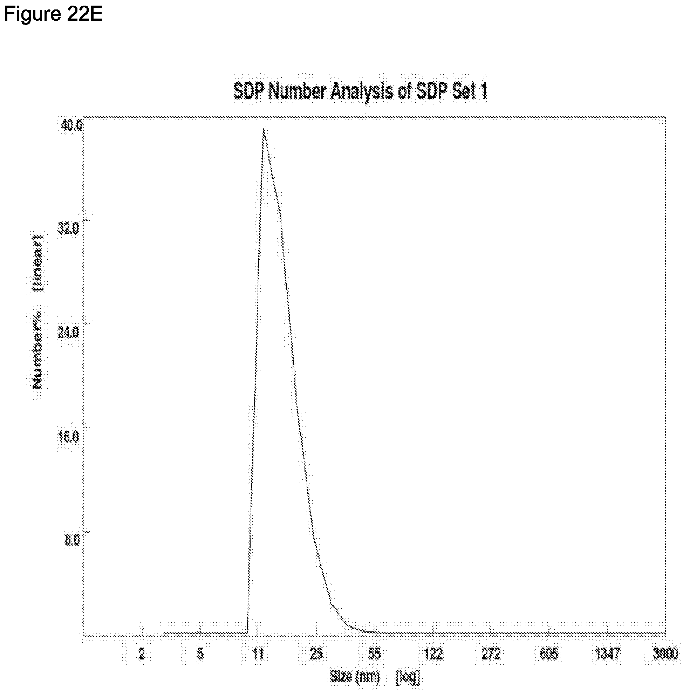

**Figures 22-25** are based on the example and method of Example 1 where MicRet has Nanocin added, in this case the Nanocin is replaced or combined with Ascorbic Acid or insulin.

**Figure 22** shows Micellar Retinol (TPGS) no Nanocin^{™} with 5% Ascorbic Acid in outer aqueous phase, low pH around 3.

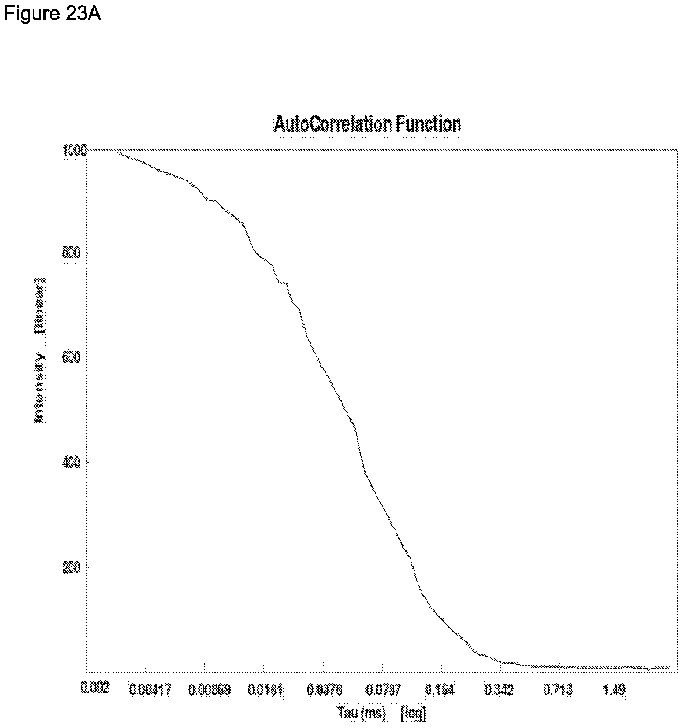

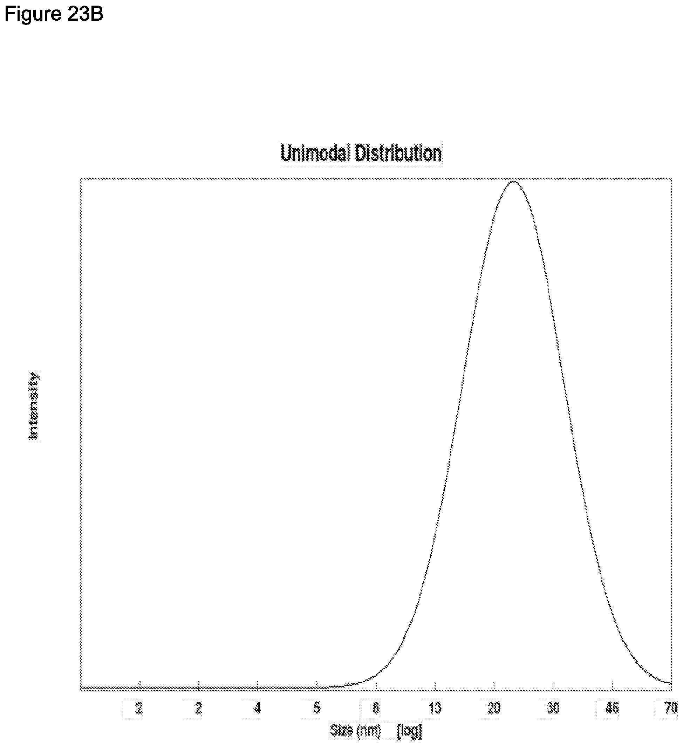

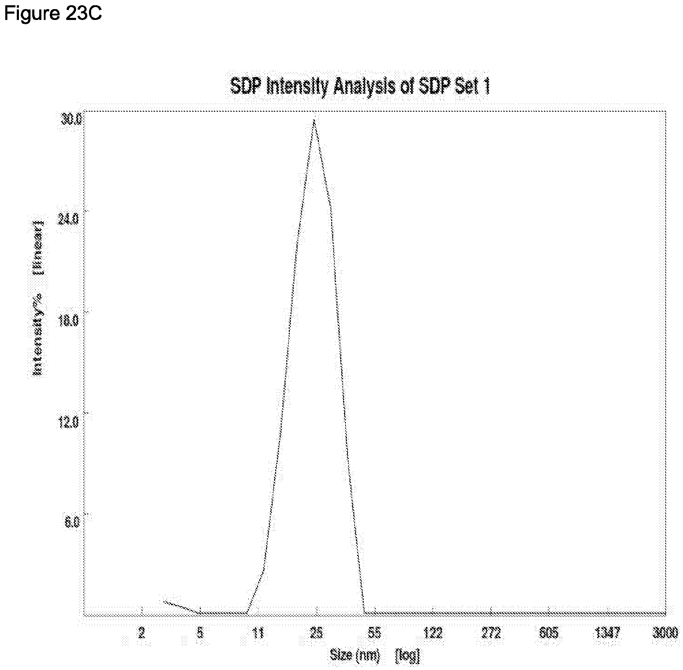

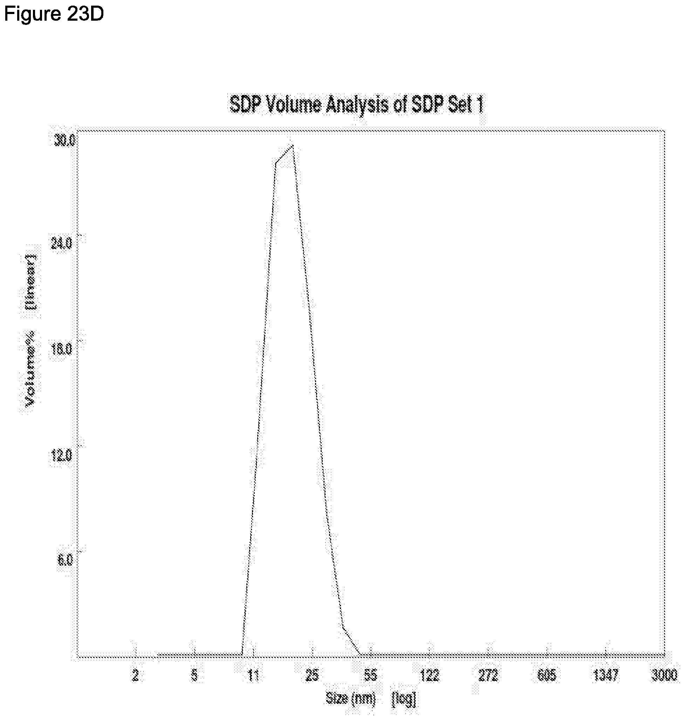

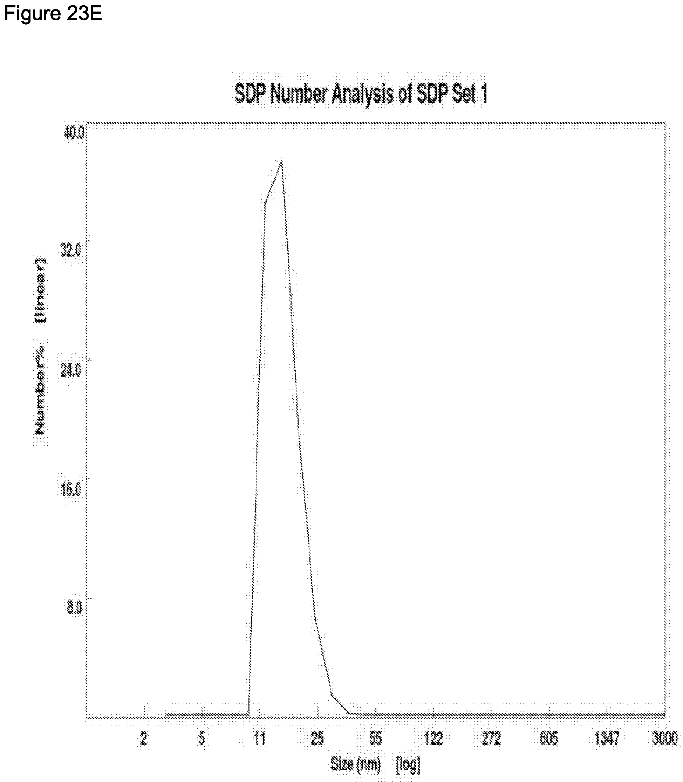

**Figure 23** shows Micellar Retinol with TPGS and insulin in outer aqueous phase.

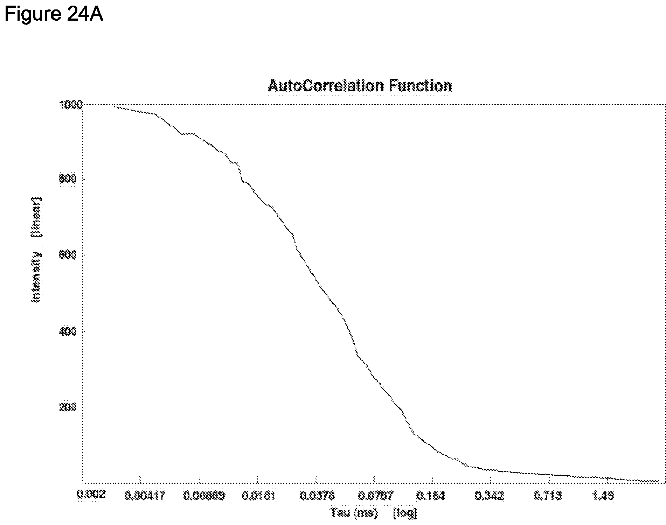

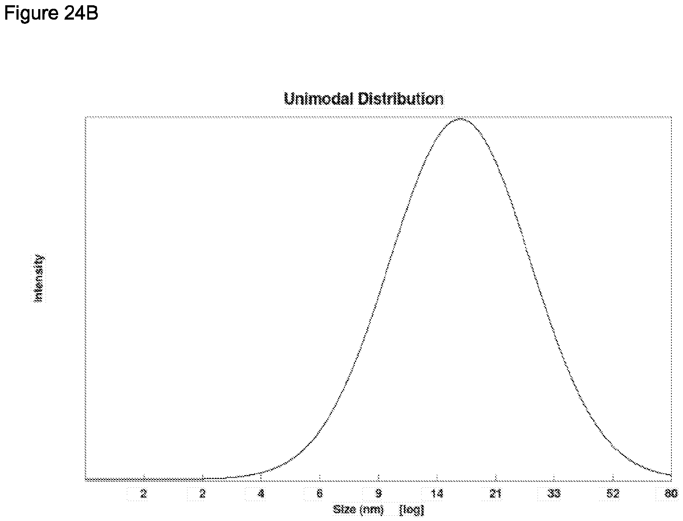

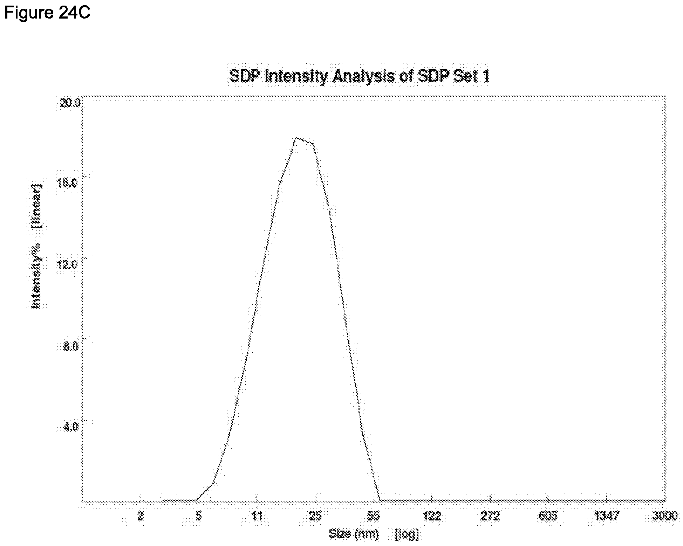

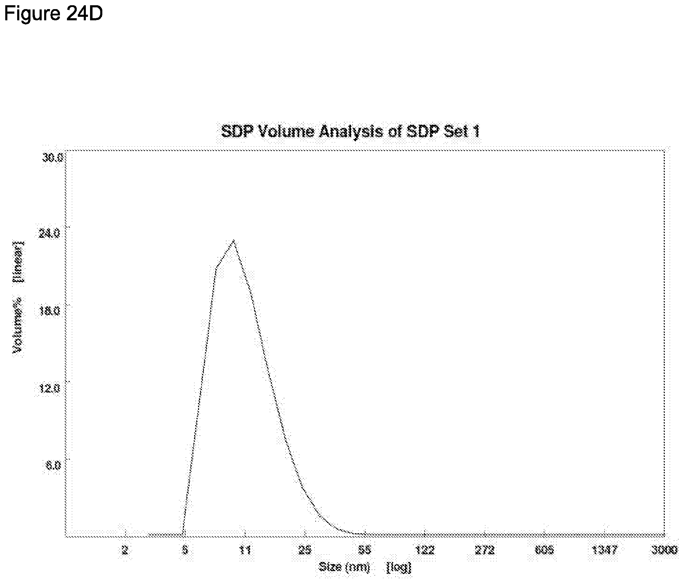

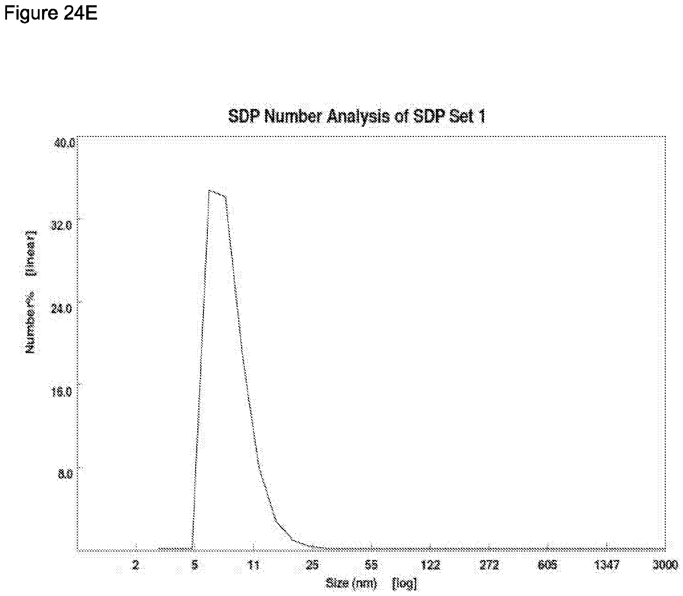

**Figure 24** shows Micellar Retinol with TPGS and Nanocin^{™} and 200 UL Ascorbic Acid in outer aqueous solution.

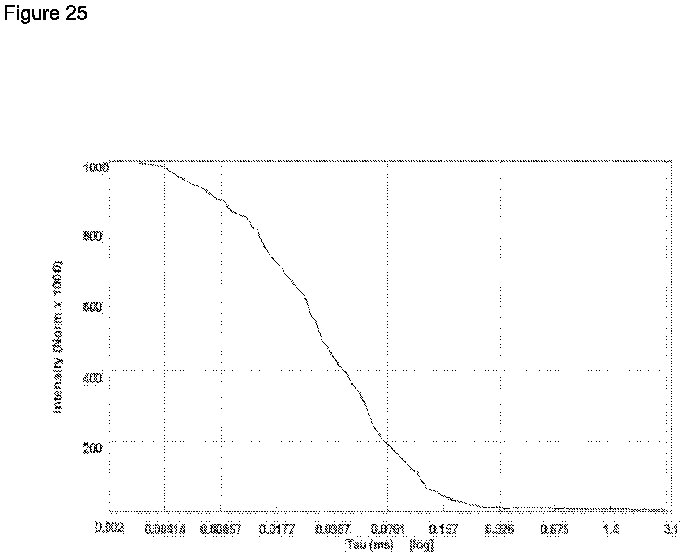

**Figure 25** shows Micellar Retinol with TPGS and Nanocin^{™} with 200µL insulin in outer aqueous phase.

### Example 9: Stability of Nanoparticles Over Time

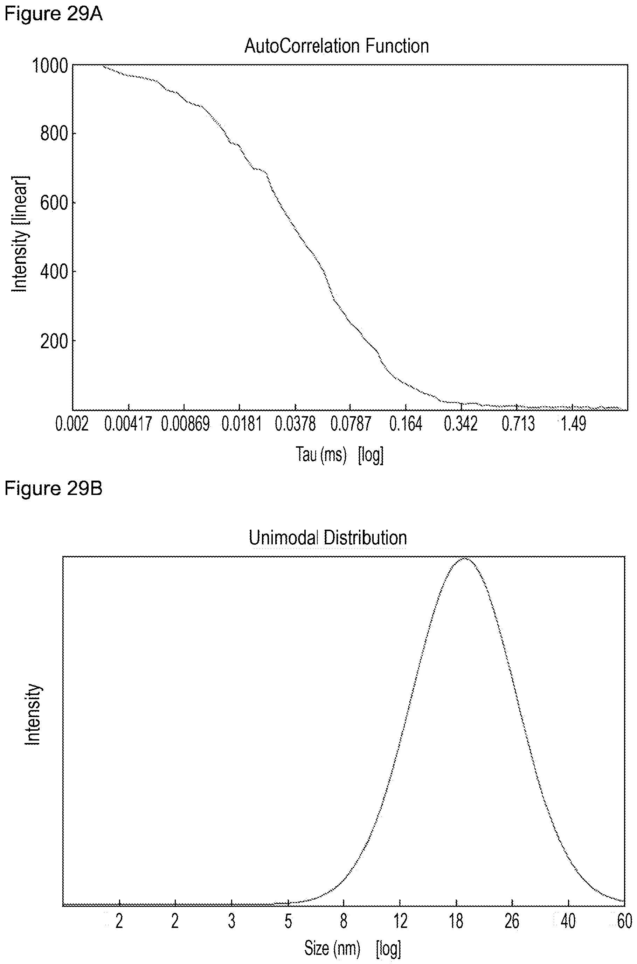

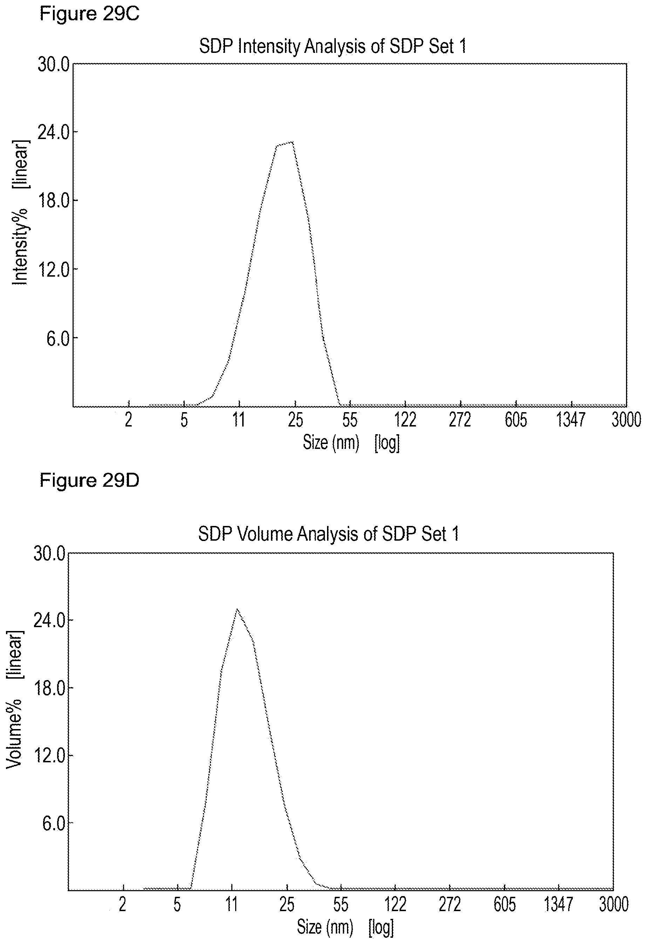

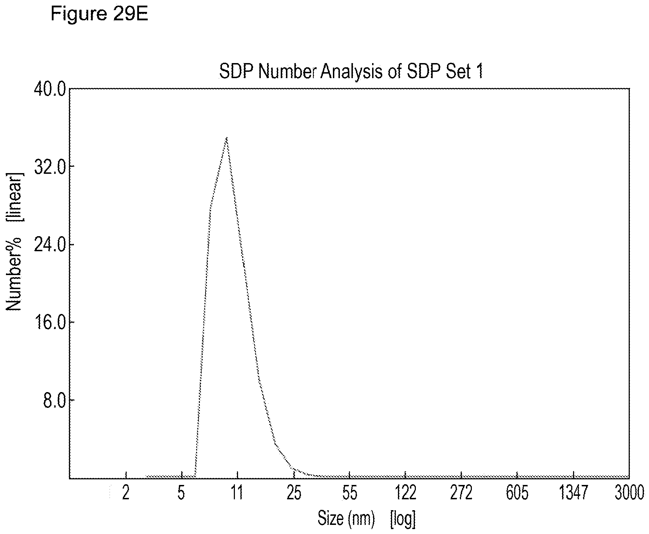

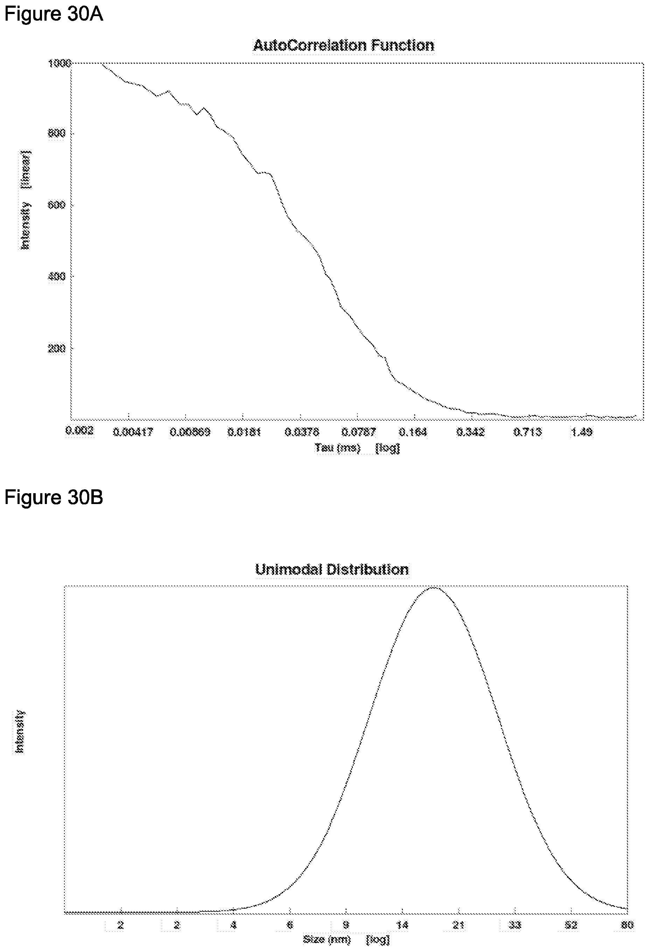

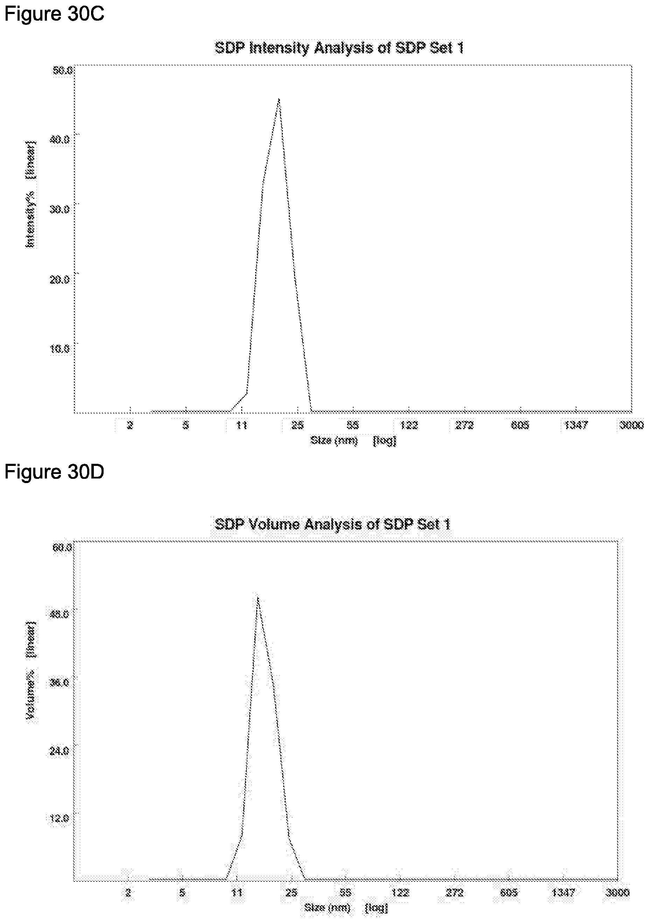

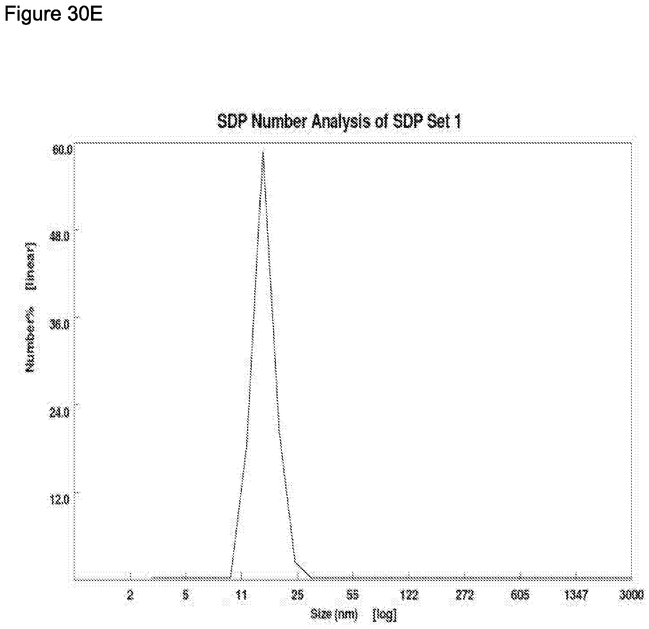

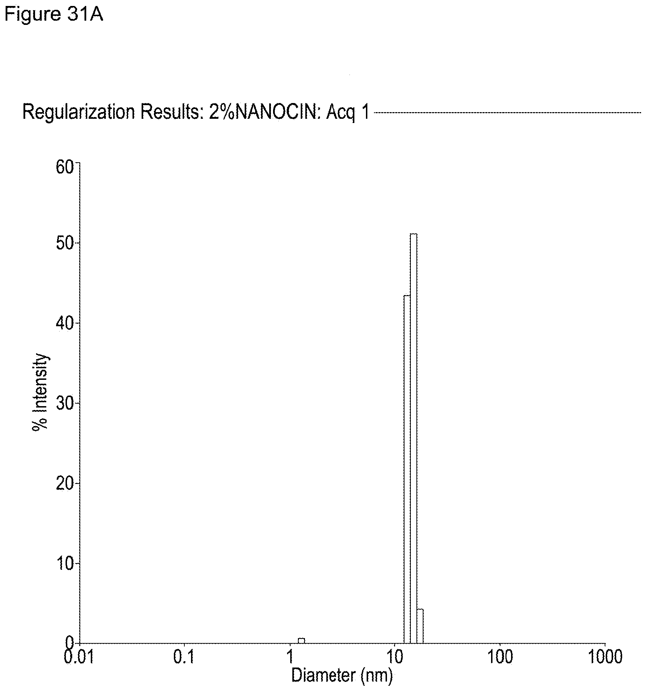

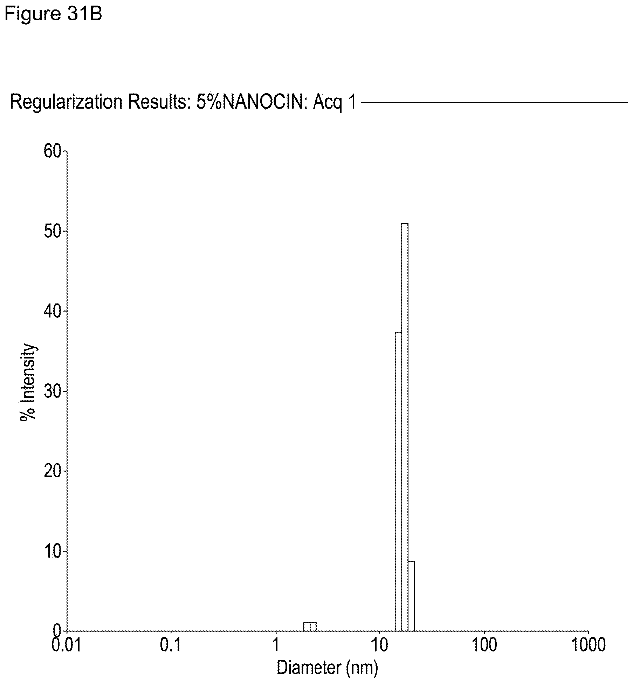

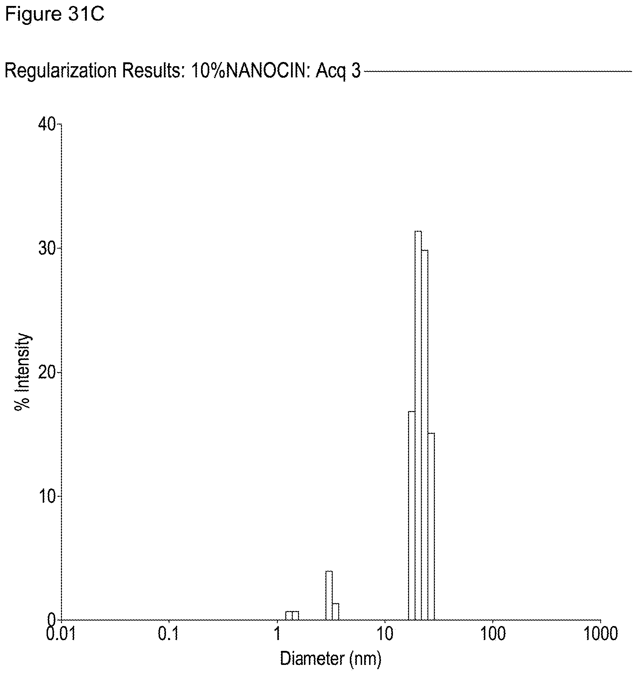

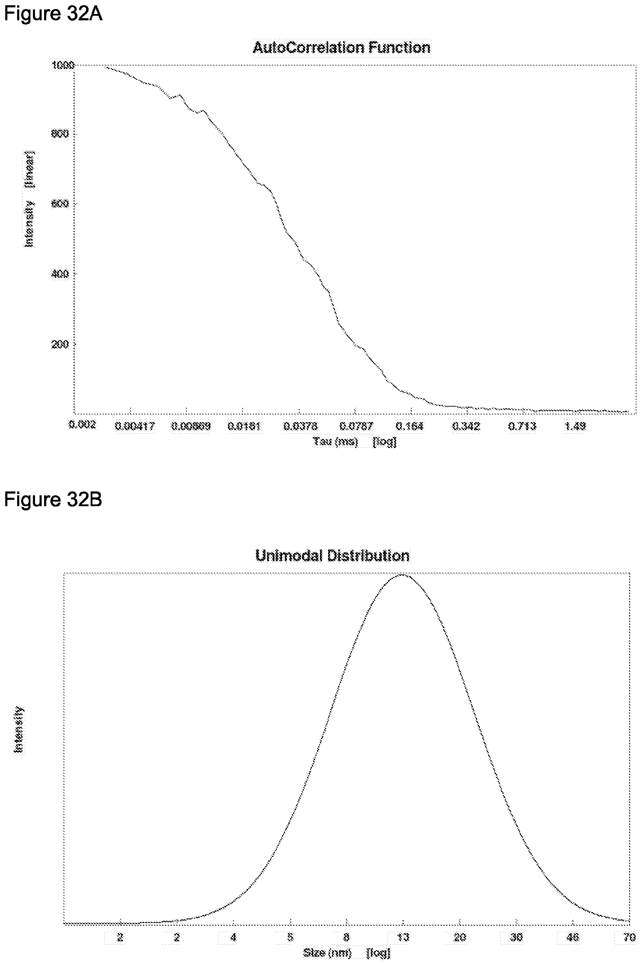

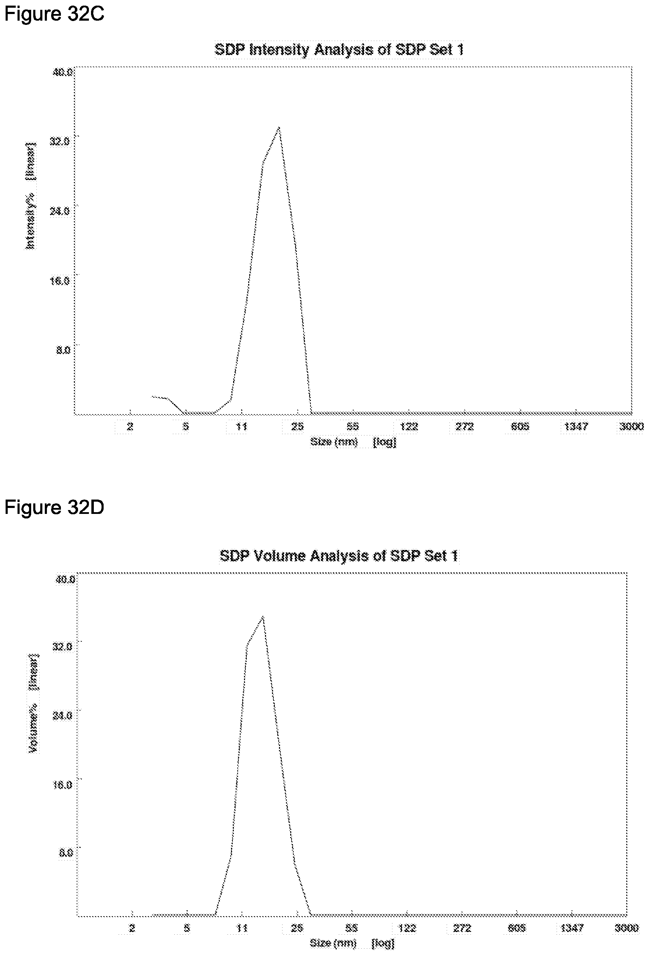

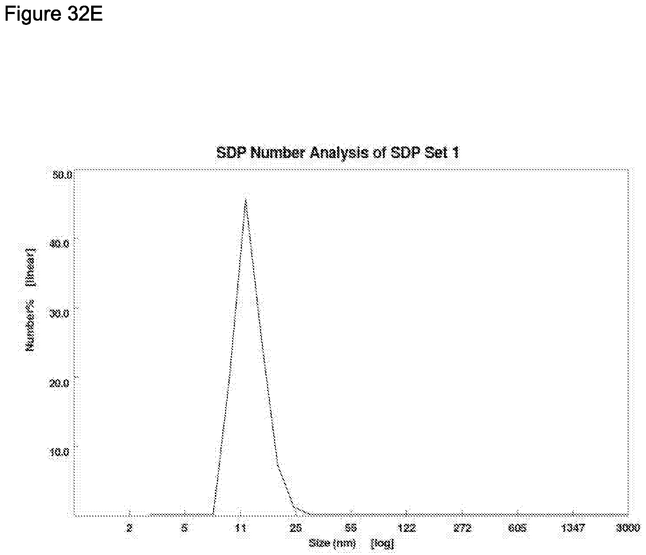

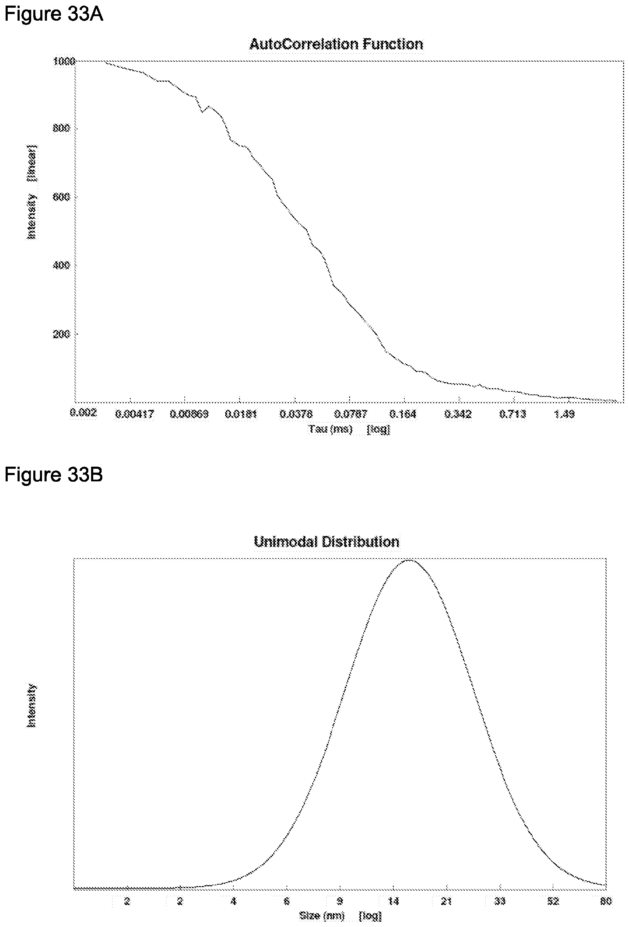

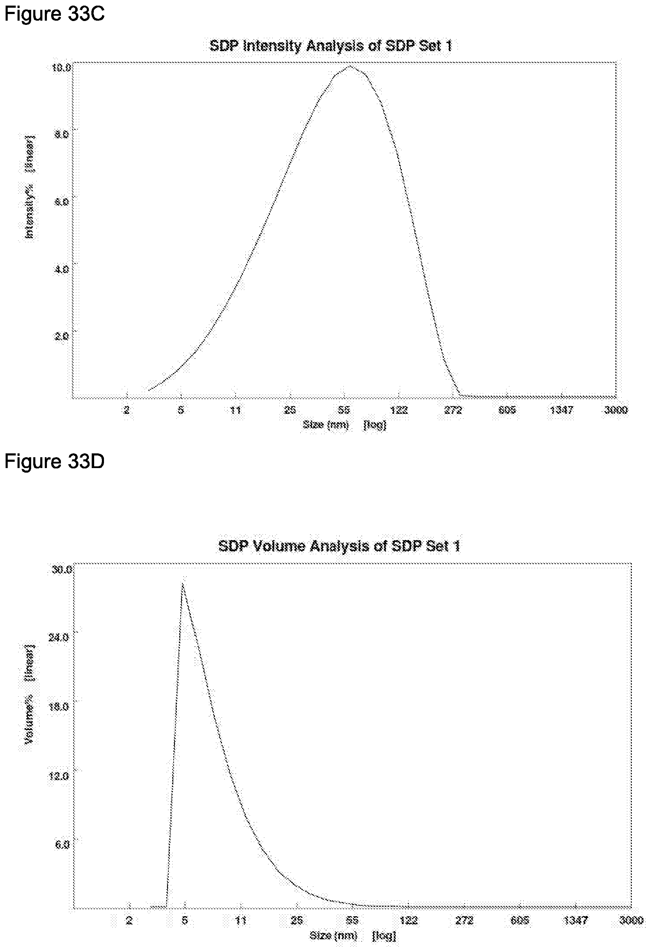

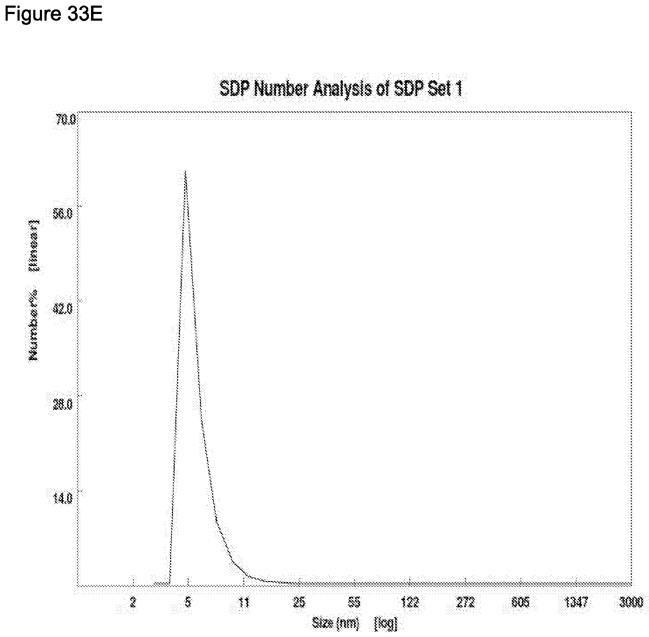

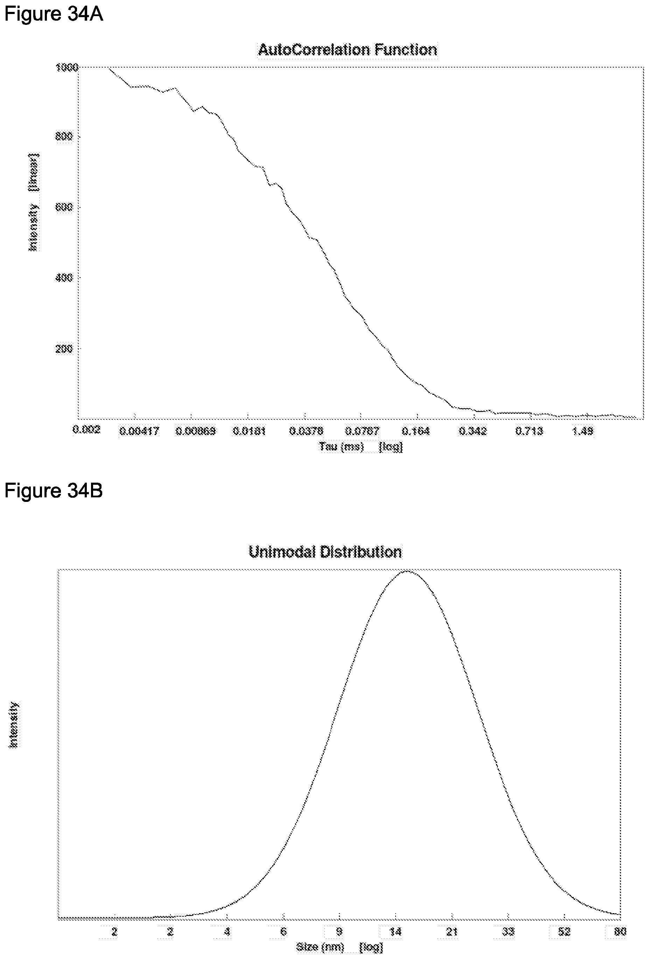

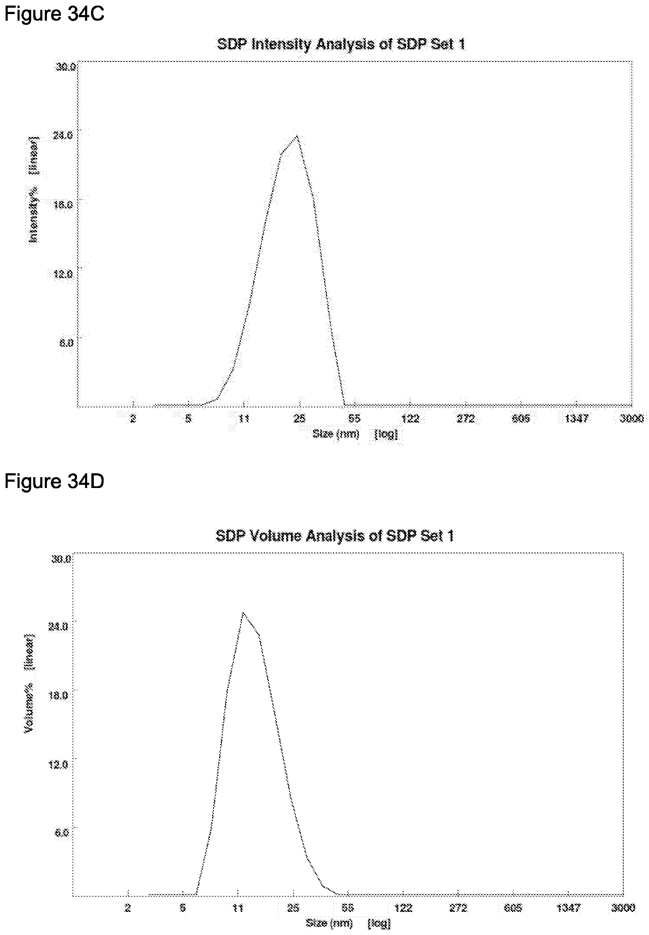

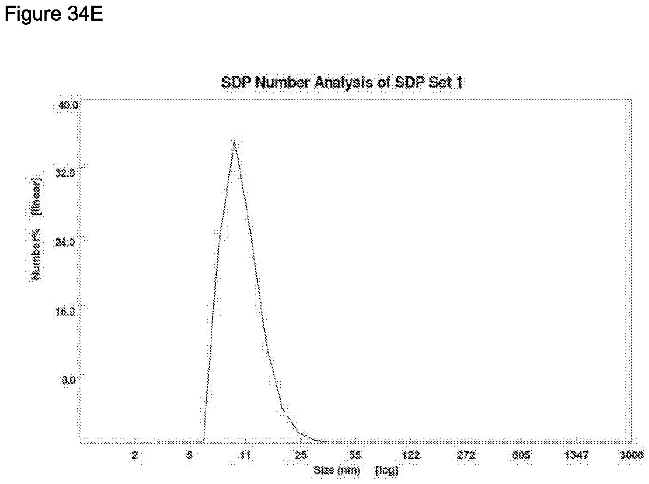

The long-term stability of micelles was assessed by 1 year storage at room temperature in darkness. TPGS micellar solutions of Retinol with different concentrations (0 %, 2%, 5%, 10%) of Nanocin^{™} was stored for 1 year at room temperature in the dark. To assess the stability of particles, measurement of absorbance with a wavelength of 405 nm was performed after initial preparation at 0 days storage and after 1 year storage at room temperature in the dark. Micelles remained stable after one year storage at room temperature, with and without Nanocin^{™} (see Figures 29 to 34).

### Example 10: Summary

The above examples demonstrate that particles made without TPGS are above 100nm and with lower loading and that not all oil soluble actives or ingredients will work for the methods outlined.

Measurements show micelles with Retinol and TPGS are small, stable and demonstrate good loading. The addition of Nanocin^{™} to these micelles gives considerably smaller nanoparticles, increased stability and increased loading than Nanocin^{™} used without TPGS.

Tocopherol and Tocotrienol give nano-micelles. Ascorbyl Palmitate requires Retinol to form micelles. Tocopherol and Tocotrienol micelles were not made using Nanocin^{™} with TPGS but the success of Nanocin addition to TPGS micelles Ascorbyl Palmitate and Retinol micelles indicates that addition of Nanocin^{™} would have a similarly dramatic effect on Tocopherol and Tocotrienol.

## Claims

1. A composition comprising micelles of aqueous d-α-tocopheryl polyethylene glycol 1000 succinate (TPGS), polyhexamethylene biguanide (PHMB) or buffered PHMB, and a bio-active therapeutic agent; wherein the micelles of the composition do not exceed 31nm.

2. The composition as claimed in claim 1, wherein the bio-active therapeutic agent is an oil-soluble active agent.

3. The composition of claim 2, wherein the oil-soluble active agent is an oil-soluble aromatic vitamin, preferably Retinol, Tocotrienol and/or Tocopherol.

4. The composition as claimed in claim 3, wherein the oil-soluble active agent is Retinol.

5. The composition of claim 4, wherein the composition further comprises Ascorbyl Palmitate.

6. The composition of claim 5, wherein the ratio of Retinol to Ascorbyl Palmitate is 50:50.

7. The composition of any one of the preceding claims, wherein the micelles do not exceed 25nm.

8. The composition of claim 7, wherein the micelles do not exceed 20nm.

9. The composition as claimed in claim 1, wherein the aqueous TPGS further comprises a water-soluble agent.

10. The composition of claim 9, wherein the water-soluble agent comprises a water-soluble active agent such as insulin, ascorbic acid or L-ascorbic acid.

11. The composition of claim 10, wherein the water-soluble agent comprises insulin.

12. A cosmetic formulation comprising the composition as claimed in any of claims 1 to 11.

13. The cosmetic formulation of claim 12, wherein the composition of micelles is in the form of a cream, lotion, gel, semi-solid, dispersion, suspension, foam, mousse or spray.

## Patentansprüche

1. Zusammensetzung, umfassend Mizellen von wässrigem D-Alpha-Tocopheryl-Polyethylenglykol-1000-Succinat (TPGS), Polyhexamethylenbiguanid (PHMB) oder gepuffertem PHMB und ein biologisch aktives therapeutisches Mittel; wobei die Mizellen der Zusammensetzung 31 nm nicht übersteigen.

2. Zusammensetzung nach Anspruch 1, wobei das biologisch aktive therapeutische Mittel ein öllöslicher Wirkstoff ist.

3. Zusammensetzung nach Anspruch 2, wobei der öllösliche Wirkstoff ein öllösliches aromatisches Vitamin, bevorzugt Retinol, Tocotrienol und/oder Tocopherol ist.

4. Zusammensetzung nach Anspruch 3, wobei der öllösliche Wirkstoff Retinol ist.

5. Zusammensetzung nach Anspruch 4, wobei die Zusammensetzung weiter Askorbylpalmitat umfasst.

6. Zusammensetzung nach Anspruch 5, wobei das Verhältnis von Retinol zu Askorbylpalmitat 50:50 ist.

7. Zusammensetzung nach einem der vorstehenden Ansprüche, wobei die Mizellen 25 nm nicht übersteigen.

8. Zusammensetzung nach Anspruch 7, wobei die Mizellen 20 nm nicht übersteigen.

9. Zusammensetzung nach Anspruch 1, wobei das wässrige TPGS weiter ein wasserlösliches Mittel umfasst.

10. Zusammensetzung nach Anspruch 9, wobei das wasserlösliche Mittel einen wasserlöslichen Wirkstoff wie Insulin, Ascorbinsäure oder L-Ascorbinsäure umfasst.

11. Zusammensetzung nach Anspruch 10, wobei das wasserlösliche Mittel Insulin umfasst.

12. Kosmetische Formulierung, umfassend die Zusammensetzung nach einem der Ansprüche 1 bis 11.

13. Kosmetische Formulierung nach Anspruch 12, wobei die Zusammensetzung der Mizellen in der Form einer Creme, Lotion, eines Gels, einer halbfesten Substanz, einer Dispersion, Suspension, Schaum, Mousse oder eines Sprays ist.

## Revendications

1. Composition comprenant des micelles de succinate 1000 de polyéthylène glycol d-α-tocophéryl (TPGS) aqueux, polyhexaméthylène biguanide (PHMB) ou PHMB mis en mémoire tampon, et un agent thérapeutique bio-actif ; dans laquelle les micelles de la composition ne dépassent pas 31 nm.

2. Composition selon la revendication 1, dans laquelle l'agent thérapeutique bio-actif est un agent actif oléosoluble.

3. Composition selon la revendication 2, dans laquelle l'agent actif oléosoluble est une vitamine aromatique oléosoluble, de préférence le rétinol, le tocotriénol et/ou le tocophérol.

4. Composition selon la revendication 3, dans laquelle l'agent actif oléosoluble est le rétinol.

5. Composition selon la revendication 4, dans laquelle la composition comprend en outre du palmitate d'ascorbyle.

6. Composition selon la revendication 5, dans laquelle le rapport du rétinol sur le palmitate d'ascorbyle est de 50/50.

7. Composition selon l'une quelconque des revendications précédentes, dans laquelle les micelles ne dépassent pas 25 nm.

8. Composition selon la revendication 7, dans laquelle les micelles ne dépassent pas 20 nm.

9. Composition selon la revendication 1, dans laquelle le TPGS aqueux comprend en outre un agent hydrosoluble.

10. Composition selon la revendication 9, dans laquelle l'agent hydrosoluble comprend un agent actif hydrosoluble tel que l'insuline, l'acide ascorbique ou l'acide L-ascorbique.

11. Composition selon la revendication 10, dans laquelle l'agent hydrosoluble comprend de l'insuline.

12. Formulation cosmétique comprenant la composition selon l'une quelconque des revendications 1 à 11.

13. Formulation cosmétique selon la revendication 12, dans laquelle la composition de micelles se présente sous la forme d'une crème, d'une lotion, d'un gel, d'une dispersion semi-solide, d'une suspension, d'une mousse, d'un produit moussant ou d'un spray.
